(19) Europäisches Patentamt
European Patent Office
Office européen des brevets

(11) Veröffentlichungsnummer: **0 001 295**
**B1**

(12) # EUROPÄISCHE PATENTSCHRIFT

(45) Veröffentlichungstag der Patentschrift: **17.03.82**

(21) Anmeldenummer: **78100994.9**

(22) Anmeldetag: **26.09.78**

(51) Int. Cl.³: **C 07 C 103/52,**
A 61 K 37/02 //C07C101/12,
C07C101/18, C07C101/20,
C07C125/06, C07D207/46,
C07D209/20, C07F7/10

(54) **Somatostatin-analoge Cyclopeptide, Verfahren zu ihrer Herstellung, pharmazeutische Präparate enthaltend diese Verbindungen, sowie ihre therapeutische Anwendung.**

(30) Priorität: **28.09.77 LU 78191**

(43) Veröffentlichungstag der Anmeldung:
**04.04.79 Patentblatt 79/7**

(45) Bekanntmachung des Hinweises auf die Patenterteilung:
**17.03.82 Patentblatt 82/11**

(84) Benannte Vertragsstaaten:
**BE CH DE FR GB NL SE**

(56) Entgegenhaltungen:
**DE - A - 2 611 779**
**DE - A - 2 656 849**

(73) Patentinhaber: **CIBA-GEIGY AG**
**Patentabteilung Postfach**
**CH-4002 Basel (CH)**

(72) Erfinder: **Rink, Hans**
**Rebenstrasse 10**
**CH-4125 Riehen (CH)**
Erfinder: **Kamber, Bruno, Dr.**
**Sonnenweg 9**
**CH-4144 Arlesheim (CH)**
Erfinder: **Sieber, Peter**
**Bachmattweg 10**
**CH-4153 Reinach (CH)**

(74) Vertreter: **Zumstein sen., Fritz, Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 295 B1

# Somatostatin-analoge Cyclopeptide, Verfahren zu ihrer Herstellung, pharmazeutische Präparate enthaltend diese Verbindungen, sowie ihre therapeutische Anwendung

Die Erfindung betrifft neue Cyclopeptide vom Somatostatin-Typ und ihre Herstellungsverfahren, sowie pharmazeutische Präparate enthaltend diese Verbindungen und Verwendung dieser Verbindungen bzw. Präparate zu therapeutischen Zwecken.

Die Erfindung betrifft insbesondere Cyclopeptide, welche die wesentlichsten Merkmale des Somatostatins, wie die Teilsequenz der Aminosäuren 5—11, oder eine äquivalente Sequenz enthalten, dabei aber schwefelfrei sind. Die erfindungsgemässen Somatostatin-analogen Cyclopeptide umfassen Verbindungen der Formel

```
┌──────────────────────────────────────┐
└ R-Phe-Phe-trp-Lys-Thr-Phe(W)-X-Y ┘        (I)
  5   6    7   8   9   10   11   1213
```

worin R für Asn, Ala oder des-R, trp für D-Trp oder L-Trp, das im Benzolring durch Halogenatome, wie insbesondere Chlor oder Fluor, oder Nitrogruppen substituiert sein kann, W für eine am Benzolring des L-Phenylalaninrestes als Substituent befindliche gegebenenfalls verätherte Hydroxylgruppe oder Halogenatom, wie insbesondere Jodatom, oder für Wasserstoff, X für den Rest einer $\omega$-Aminoniederalkan-(mono oder di)-carbonsäure oder des-X und Y für den Rest einer $\omega$-Aminoniederalkan-(mono der di)-carbonsäure oder des-Y steht, mit Ausnahme einer Verbindung, worin R für des-R, trp für D-Trp oder L-Trp, W für Wasserstoff, eines der Symbole X and Y für des-X bzw. des-Y und das andere für der Rest der 7-Aminoheptansäure steht, sowie Säureadditionssalze und Komplexe davon.

Die im Benzolring des Tryptophan$^8$-Rests gegebenenfalls vorhandenen Halogenatome befinden sich vorzugsweise in der 5-Stellung, die Nitrogruppe vorzugsweise in der 6-Stellung; besonders zu nennen sind die vom 5-Brom-L-tryptophan, 5-Fluor-D-tryptophan und 6-Nitro-D-tryptophan abgeleiteten Reste. -Der Substituent W des L-Phenylalanin$^{11}$-Restes befindet sich vorzugsweise in der p-Stellung des Benzolringes; besonders bevorzugt, neben dem unsubstituierten L-Phenylalanin-Rest, sind auch der p-Jod-L-phenylalanin und p-Hydroxy-L-phenylalanin-Rest. Die Hydroxygruppe des letzteren Restes (d.h. des L-Tyrosin-Restes) kann auch in einer verätherten Form vorliegen; dazu sind Niederalkyle, sowie verschiedene in der Peptid-Chemie übliche Aether-bildende Hydroxyl-Schutzgruppen, z.B. die weiter unten genannten, geeignet; besonders bevorzugt ist die tert-Butylgruppe, und der bevorzugte Aminosäure$^{11}$-rest leitet sich vom p-tert-Butoxyphenylalanin (d.h. Tyrosin-tert-butyläther) ab.

Der Rest einer $\omega$-Aminoniederalkanmonocarbonsäure ist vorzugsweise ein Rest der Formel

$$—NH—(CH_2)_n—CO—$$

worin n eine ganze Zahl von 1 bis 7, insbesondere 2—4, bedeutet. Besonders bevorzugt ist der Rest der $\gamma$-Aminobuttersäure, der mit dem Symbol Gaba bezeichnet wird.

Der Rest einer $\omega$-Aminoniederalkandicarbonsäure leitet sich vorzugsweise von einem Rest der Formel

```
—NH—CH—(CH2)n-1—CO—
     |
     COOH
```

ab, worin n die obenstehende Bedeutung hat und die Carboxylgruppe auch in einer funktionell abgewandelten Form, z.B. als ein Ester oder Amid, wie ein in der Peptidchemie üblicher Ester oder ein unsubstituiertes Amid, vorliegen kann. Besonders bevorzugt ist ein Rest der L- oder insbesondere der D-Glutaminsäure der Formel

```
—NH—CH—(CH2)2—CO—
     |
     COOH
```

oder ein entsprechender Rest, in welchem sich anstelle der Carboxylgruppe die Carbamoylgruppe befindet.

Im Einklang mit dem üblichen nomenklatorischen Gebrauch bedeutet die Vorsilbe "des" das Fehlen eines damit bezeichneten Symbols; so besagt z.B. der Ausdruck "des-X", dass das Symbol X die Bedeutung $[X]_m$, worin m = 0, hat und in der ensprechenden Formel auszulassen ist.

Die bevorzugten erfindungsgemässen Somatostatin- Analogen sind solche, worin R vorzugsweise für Asn oder des-R steht, der Tryptophan$^8$-Rest die D-Konfiguration hat und —X—Y— zusammen -$[Gaba]_p$-, worin p gleich 0, 1 oder 2 ist, bedeutet. Als besonders bevorzugt sind hervorzuheben die Verbindungen der Formel

```
┌──────────────────────────────────────────────┐
└ Asn-Phe-Phe-[D-trp]-Lys-Thr-Phe-[Gaba]p ┘        (IA)
   5    6   7     8     9   10  11
```

worin p = 2 oder insbesondere p = 1 ist. Alle diese bevorzugten Somatostatin-Analogen können auch in Form von Säureadditionssalzen oder Komplexen vorliegen.

Als Säureadditionssalze kommen besonders physiologisch verträgliche Salze mit üblichen therapeutisch anwendbaren Säuren in Betracht; von den anorganischen Säuren sind die Halogenwasserstoffsäuren, wie die Chlorwasserstoffsäure, aber auch Schwefelsäure und Phosphor- bzw. Pyrophosphorsäure zu nennen, von den organischen Säuren sind es in erster Linie die Sulfonsäuren, wie die Benzol- oder p-Toluol-Sulfonsäure, oder Niederalkansulfonsäuren, wie Methansulfonsäure, ferner auch Carbonsäuren, wie Essigsäure, Milchsäure, Palmitin- und Stearinsäure, Aepfelsäure, Weinsäuren, Ascorbinsäure und Zitronensäure.

Unter Komplexen sind die in ihrer Struktur noch nicht völlig abgeklärten Verbindungen zu verstehen, die beim Zusatz gewisser anorganischer oder organischer Stoffe zu Peptiden entstehen und diesen eine verlängerte Wirkung verleihen. Solche Stoffe sind beispielsweise beschrieben für ACTH und andere adrenocorticotrop wirksame Peptide. Zu nennen sind z.B. anorganische Verbindungen, die sich von Metallen wie Calcium, Magnesium, Aluminium, Cobalt und insbesondere von Zink ableiten, vor allem schwerlösliche Salze, wie Phosphate, Pyrophosphate und Polyphosphate, sowie Hydroxyde dieser Metalle, ferner Alkalimetallpolyphosphate, z.B. "Calgon N", "Calgon 322", "Calgon 188" oder "Polyron B 12". Organische Stoffe, die eine Verlängerung der Wirkung hervorrufen, sind beispielsweise nicht antigene Gelatine-Arten, z.B. Polyoxygelatine, Polyvinylpyrrolidon und Carboxymethylcellulose, ferner Sulfonsäure- oder Phosphorsäureester von Alginsäure, Dextran, Polyphenolen und Polyalkoholen, vor allem Polyphloretinphosphat und Phytinsäure, sowie Polymerisate und Copolymerisate von basischen oder vor allem sauren Aminosäuren, z.B. Protamin oder Polyglutaminsäure.

Wenn nicht anders angegeben, bezeichnet der Begriff "nieder", wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

Die neuen erfindungsgemässen Cyclopeptide weisen eine physiologische Wirkung auf, die im Charakter und Intensität der Wirkung des Somatostatins äquivalent ist. Sie können deshalb in ähnlichen therapeutischen Indikationen wie dieses, z.B. zur Behandlung von Funktionsstörungen, in denen die Sekretion des Wachstumshormons oder des Insulins und Glucagons übernormal hoch ist, wie bei Acromegalie oder juvenilem Diabetes, mit Vorteil angewendet werden.

Somatostatin, ein cyclisches Tetradecapeptid der Formel

H-Ala-Gly-Cys-Lys-Asn-Phe-Phe-Trp-Lys-Thr-Phe-Thr-Ser-Cys-OH
1 2 3 4 5 6 7 8 9 10 11 12 13 14

[Science 179,77 (1973)] hemmt bekanntlich die Hypophysengesteurte Sekretion des Wachstumshormons (Somatotropin). Ausserdem hemmt es die sekretorische Aktivität des endocrinen Pancreas, wie die Sekretion von Insulin und Glucagon.

Diese wertvollen Eigenschaften können beim Somatostatin selber nicht zur vollen praktischen Anwendung gelangen, da einer technischen Synthese dieser Verbindung ihre komplizierte chemische Struktur im Wege steht. Neben der hohen Anzahl von Aminosäuren ist auch die Anwesenheit von Cystin von besonderem Nachteil: wegen ihres Schwefelgehaltes verhindert diese Aminosäure die Anwendung der äusserst vorteilhaften hydrogenolytisch abspaltbaren Schutzgruppen. Dieser Nachteil wurde später beseitigt, indem man in DE—A— 26 56 849 und DE—A—26 11 779 gezeigt hat, dass man ohne eine nennenswerte Beeinträchtigung der günstigen physiologischen Wirkung nicht nur die zwei ersten, ausserhalb des Peptid-Ringes stehenden Aminosäuren auslassen, sondern insbesondere im Peptid-Ring den Schwefelhaltigen Cystin-Rest durch einen analog gebauten, aber schwefelfreien Rest einer 4—9 C-Atome enthaltenden linearen $\omega$-Aminoniederalkan-(mono bzw. di)-carbonsäure ersetzen kann.—Abgesehen vom besprochenen Nachteil weisen 7 Aminosäuren des Somatostatin-Ringes weitere funktionelle Gruppen in der Seitenkette auf; diese Gruppen benötigen einerseits einen besonderen vorübergehenden Schutz, andererseits erhöhen sie beträchtlich die Gefahr der Razemisierung und komplizieren somit noch weiter die Bedingungen der Synthese.

Ueberraschenderweise wurde nun festgestellt, dass man im Molekül des Somatostatins mehrere dieser substituierten Aminosäuren, insbesondere auch Cystin, auslassen oder durch einfache, optisch inaktive Aminosäuren ersetzen kann, wobei die physiologische Aktivität erhalten bleibt.

Der technische Vorteil für die Synthese von Somatostatin-aktiven Stoffen ist schon daraus klar ersichtlich, dass für die Synthese von Somatostatin 13 optisch aktive Aminosäuren (davon 7 mit einer weiteren geschützten funktionellen Gruppe) als Bausteine notwendig sind, wogegen man bei den erfindungsgemässen Verbindungen mit höchstens 7 optisch aktiven Aminosäuren (davon nur 2 mit einer weiteren geschützten funktionellen Gruppe) auskommt.

Einen solchen technischen Vorteil weisen auch die in EP—A—53 (Anmeldungsnummer 78100095.5) beschriebenen Cycloheptapeptide auf, die sich von Aminosäuren 6 bis 11 des Somatostatins, oder einigen Austauschsäuren davon, ableiten, welche jeweils durch 7-Aminoheptansäure zum Cyclus verknüpft sind. Durch dieses Merkmal unterscheiden sich diese Verbindungen von den analog gebauten Vertretern der vorliegenden Erfindung, d.h. von den Verbindungen der Formel I, in welchem R für des-R steht.

Die erfindungsgemässen Verbindungen können nach an sich bekannten Methoden hergestellt werden. Insbesondere werden sie erhalten, indem man aus einer Verbindung der Formel

$$\llcorner\text{R-Phe-Phe-trp-Lys(A)-Thr(B)-Phe(W)-X-Y}\lrcorner \quad \text{(II)},$$

worin R, W, X, Y und trp die obgenannten Bedeutungen haben, A eine $\varepsilon$-Aminoschutzgruppe oder Wasserstoff und B eine Hydroxylschutzgruppe oder Wasserstoff bedeutet, wobei nur eines der Symbole A und B für Wasserstoff stehen kann, die Schutzgruppe(n) abspaltet.

Als $\varepsilon$-Amino-Schutzgruppe können alle in der Peptid-Chemie üblichen Amino-Schutzgruppen verwendet werden, wie sie in den entsprechenden Nachschlagewerken, z.B. in Houben-Weyl: Methoden der organischen Chemie; 4. Auflage, Band 15/I, E. Wünsch (Herausgeber): Synthese von Peptiden. (Georg Thieme Verlag, Stuttgart, 1974) zusammenfassend beschrieben werden. Bevorzugt sind acidolytisch abspaltbare Gruppen, wie in erster Linie die tert-Butoxycarbonyl-gruppe und analoge Gruppen, z.B. die tert-Amyloxycarbonyl-, Isopropyloxycarbonyl-, Diisopropylmethoxycarbonyl-, Allyloxycarbonyl-, Cyclopentyloxycarbonyl-, Cyclohexyloxycarbonyl-, d-Isobornyloxycarbonyl- und Adamantyloxycarbonylgruppe, sowie auch Gruppen des Aralkyl-Typs, wie Benzhydryl und Triphenylmethyl (Trityl), oder bestimmte Aralkoxycarbonyl-Gruppen des 2-(p-Biphenyl-yl)-2-propyloxycarbonyl-Typs, die in der Schweizer Patentschrift 509 266 beschrieben sind.

Ferner kann man aber auch reduktiv oder basisch abspaltbare Amino-Schutzgruppen verwenden, z.B. insbesondere die Benzyloxycarbonyl-Gruppe und Benzyloxycarbonyl-Gruppen, die im aromatischen Teil durch Halogenatome, Nitrogruppen, Niederalkoxygruppen und/oder Niederalkylreste substituiert sind, wie die p-Chlor- und p-Brombenzyloxycarbonyl-, p-Nitrobenzyloxycarbonyl-, p-Methoxybenzyloxycarbonyl-, p-Tolyloxycarbonyl-Gruppe, oder aber die Isonicotinyloxycarbonylgruppe, weiter auch Acylgruppen, wie p-Toluolsulfonyl, Benzolsulfenyl, o-Nitrobenzolsulfenyl bzw. auch Formyl, Trifluoracetyl oder Phthaloyl.

Eine besonders vorteilhafte $\varepsilon$-Amino-Schutzgruppe ist eine Aethoxycarbonylgruppe, die in $\beta$-Stellung eine mit drei Kohlenwasserstoffresten substituierte Silylgruppe, wie Triphenylsilyl-, Dimethylbutyl-silyl- oder vor allem Trimethylsilylgruppe, trägt. Eine solche $\beta$-Trihydrocarbylsilyl)-äthoxycarbonylgruppe, wie eine $\beta$-(Triniederalkylsilyl)-äthoxycarbonyl-, z.B. insbesondere die $\beta$-(Trimethylsilyl)-äthoxycarbonylgruppe, bildet mit der zu schützenden $\varepsilon$-Aminogruppe eine entsprechende $\beta$-Trihydrocarbylsilyl-äthoxycarbonylaminogruppe (z.B. die $\beta$-Trimethylsilyläthoxycarbonylaminogruppe), welche gegen die Bedingungen der sauren Hydrolyse und der Hydrogenolyse beständig ist, aber unter ganz spezifischen, sehr milden Bedingungen sich durch Einwirkung von Fluoridionen abspalten lässt. In dieser Beziehung verhält sie sich analog wie die weiter unten als Carboxyl-Schutzgruppe beschriebene $\beta$-Silyläthylestergruppe. (Diese Aehnlichkeit muss bei der Synthese besonders berücksichtigt werden: bis auf Einzelfälle schliesst die Anwendung einer dieser Schutzgruppen die gleichzeitige Anwendung der anderen Schutzgruppe aus.) Weitere Einzelheiten sind weiter unten beim Schutz der Carboxylgruppe durch $\beta$-Silyläthylester angegeben.

Als Hydroxylschutz-Gruppe können alle zu diesem Zwecke in der Peptid-Chemie gebräulichen Gruppen verwendet werden, vgl. das oben zitierte Werk (Houben-Weyl). Bevorzugt sind acidolytisch abspaltbare Gruppen, wie 2-Tetrahydropyranyl und ganz besonders tert-Butyl, sowie auch tert-Butoxycarbonyl. Ferner kann man aber auch reduktiv oder basisch abspaltbare Hydroxyl-Schutzgruppen verwenden, z.B. Benzyl- und Benzyloxycarbonylgruppen, die im aromatischen Teil durch Halogen, Nitro und/oder Niederalkoxy substituiert sein können, bzw. die Niederalkanoylreste, wie Acetyl oder Aroylreste, wie Benzoyl.

Vorzugsweise werden die Schutzgruppen A und B so gewählt, das sie unter ähnlichen Bedingungen abspaltbar sind; besonders bevorzugt sind dabei die bereits hervorgehobenen acidolytisch abspaltbaren Gruppen. Die Abspaltung beider Schutzgruppen erfolgt dann vorteilhaft in einer einzigen Operation; es können jedoch auch Gruppen verschiedener Art verwendet werden und jede einzeln abgespalten werden.

Die Abspaltung der Schutzgruppen erfolgt in der allgemein bekannter Weise; die saure Hydrolyse (Acidolyse) wird z.B. mittels Trifluoressigsäure, Salzsäure oder Fluorwasserstoff, bei säureempfindlichen Schutzgruppen auch mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder Hexafluoraceton, durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt. Die Isonicotinyloxycarbonylgruppe wird vorzugsweise durch Zink-Reduktion abgespalten.

Die erfindungsgemässen Endstoffe werden, je nach der Art der Isolierung, als Basen oder als Säureadditionssalze erhalten; diese können nachträglich in an sich bekannter Weise ineinander umgewandelt werden.

Auch die Bildung der oben erwähnten Komplexe erfolgt nach bekannten oder diesen äquivalenten Methoden.

Komplexe mit anorganischen Stoffen wie schwerlöslichen Metall-, z.B. Aluminium- oder Zinkverbindungen werden vorzugsweise in analoger Weise wie für ACTH bekannt, z.B. durch Unsetzung mit

einem löslichen Salz des betreffenden Metalls, z.B. Zinkchlorid oder Zinksulfat, und Ausfällung mit einem Alkalimetallphosphat und/oder -hydroxyd hergestellt. Komplexe mit organischen Verbindungen wie Polyoxygelatine, Carboxymethylcellulose, Polyvinylpyrrolidon, Polyphloretinphosphat, Polyglutaminsäure etc. werden durch Mischen dieser Substanzen mit dem Peptid in wässeriger Lösung erhalten. In gleicher Weise können auch unlösliche Verbindungen mit Alkalimetallpolyphosphaten hergestellt werden.

Die Verbindungen der oben charakterisierten Formel II sind neu und gehören auch zum Gegenstand der Erfindung. Sie werden erhalten, indem man ein entsprechendes lineares Peptid der Formel

H—[II']—C (III)

worin II' einen der Formel II entsprechenden Rest darstellt, in welchem die amidische Bindung zwischen zwei beliebigen benachbarten Aminosäureresten des Peptidringes unterbrochen ist, und C für eine freie Hydroxylgruppe, eine durch eine Aktivierungsgruppe abgewandelte Hydroxylgruppe oder die Hydrazinogruppe —NH—$NH_2$ steht, cyclisiert.

Unter den linearen Peptiden der Formel III sind diejenigen bevorzugt, worin mindestens einer der Reste X und Y im Rest [II'] eine endständige Aminosäure bildet. Diese bevorzugten Ausgangsstoffe sind durch die Formeln

H-X-Y-R-Phe-Phe-trp-Lys(A)-Thr(B)-Phe-C (IIIa)

H-Y-R-Phe-Phe-trp-Lys(A)-Thr(B)-Phe-X-C (IIIb)

und insbesondere

H-R-Phe-Phe-trp-Lys(A)-Thr(B)-Phe-X-Y-C (IIIc)

charakterisiert, worin A, B, C, trp, R, X und Y die obgenannten Bedeutungen haben. Ganz besonders bevorzugt sind Verbindungen der Formeln IIIa-c, worin A für eine Amino-Schutzgruppe, vor allem eine acidolytisch abspaltbare Amino-Schutzgruppe, B für eine Hydroxylschutzgruppe, vor allem eine acidolytisch abspaltbare Hydroxylschutzgruppe und C für eine freie Hydroxylgruppe steht.

Eine durch das Symbol C dargestellte funktionelle Gruppe ergänzt die Carbonylgruppe des C-terminalen Aminosäure-Rests und bildet zusammen mit ihr eine freie Carboxylgruppe, eine aktivierte Estergruppe, beziehungsweise die Carbazoylgruppe.

Die Aktivierungsgruppe, durch welche die Hydroxylgruppe abgewandelt ist, ist insbesondere eine solche, die den aktivierten Ester von N-Hydroxysuccinimid, 1-Hydroxybenzotriazol, N,N'-Dicyclohexylisoharnstoff, 2,4,5-Trichlorphenol, 2-Nitrophenol, 4-Nitrophenol, Pentachlorphenol oder Pentafluorphenol bildet, aber auch eine andere aus der Peptidchemie bekannte Aktivierungsgruppe dieser Art, vgl. Houben-Weyl, Band 15/II.

Die erfindungsgemässe Cyclisierung der linearen Peptide der Formel III erfolgt in an sich bekannter Weise mittels üblicher, zur Ausbildung der Amid-Bindung gebräuchlicher Kupplungsmethoden, wobei aber die peptidischen Ausgangsstoffe in einer sehr niedrigen Konzentration eingesetzt werden, um den Verlauf der Kupplung zugunsten der intramolekularen Cyclisierung auf Kosten der intermolekularen Polykondensation zu verschieben.

Die linearen Peptide werden mit Vorteil in einer etwa $1.10^{-5}$-molaren bis etwa $1.10^{-3}$-molaren, vorzugsweise einer etwa $1.10^{-4}$-molaren Konzentration eingesetzt, die einer Gewicht/Volumen-Konzentration von etwa 0,01 bis 1,0%, vorzugsweise 0,1% entspricht. Eine entsprechende Verdünnung kann im Reaktionsgemisch vom Anfang an eingestellt werden, oder aber durch langsames Eintropfen des Ausgangsstoffes und gegebenenfalls der übrigen Reagentien in das Reaktionsgemisch fortlaufend hergestellt werden.

Vorzugsweise erfolgt die Cyclisierung, indem man bei einer oben angegebenen Anfangskonzentration a) einen Ausgangsstoff der Formel III, worin C eine freie Hydroxylgruppe bedeutet und in welchem sowohl die $\varepsilon$-Aminogruppe des Lysinrests wie die Hydroxylgruppe des Threoninrests geschützt sind, mit einem Carbodiimid, gegebenenfalls in Anwesenheit einer Aktivester-bildenden Komponente, behandelt, oder b) einen Ausgangsstoff der Formel III, worin C eine zum aktivierten Ester abgewandelte Hydroxylgruppe darstellt und die endständige Aminogruppe in protonierter Form vorliegt, wobei mindestens die $\varepsilon$-Aminogruppe des Lysinrests geschützt ist, mit einer organischen Base umsetzt, oder c) einen Ausgangsstoff der Formel III, worin C die Gruppe —$NHNH_2$ bedeutet und mindestens die $\varepsilon$-Aminogruppe des Lysinrests geschützt ist, zunächst unter sauren Bedingungen mit salpetriger Säure oder einem Niederalkylester davon behandelt und nachher bei einer oben erwähnten niedrigen Konzentration mit überschüssiger organischer Base cyclisiert.

Die Cyclisierung führt man in geeigneten Lösungsmitteln durch; beispielsweise sind Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie auch Chloroform und Methylenchlorid und Gemische davon zu nennen.

Bei der Verfahrensvariante a) wird die Cyclisierung durch ein Carbodiimid, vorzugsweise N,N'-Dicyclohexylcarbodiimid, das man mit Vorteil im Ueberschuss anwendet, herbeigeführt; es ist anzunehmen, dass dabei der Ausgangsstoff der Formel III mit freier Carboxylgruppe primär in einen aktivierten Ester des Dicyclohexylisoharnstoffes (bzw. eines analogen Isoharnstoffes) übergeht und dieser *in situ* gebildete aktive Ester gleich weiterreagiert. Auf eine intermediäre Bildung eines aktiven Esters ist zweifellos die Zugabe einer Aktivester-bildenden Komponente als Hilfsreagens zurückzuführen; zu diesem Zwecke können in der Peptidchemie übliche Aktivester-bildende Komponenten dienen, wie insbesondere 2,4,5-Trichlorphenol, 2- oder 4-Nitrophenol, Pentachlor- und Pentafluorphenol, aber vor allem N-Hydroxyverbindungen, wovon als besonders vorteilhaft N-Hydroxysuccinimid, N-Hydroxypiperidin und vor allem 1-Hydroxybenzotriazol zu erwähnen sind. Die Arbeitstemperatur bei dieser Variante beträgt im allgemeinen 0—70°, vorzugsweise 35—55°.

Bei der Variante b), die mit fertigen Aktivestern, insbesondere den bereits hervorgehobenen, arbeitet, erfolgt die Cyclisierung spontan, als die endständige Aminogruppe durch die organische Base entprotonisiert wird. Die verwendeten Basen sind vorzugsweise quaternäre oder vor allem tertiäre Amine, z.B. Triäthylamin oder N-Aethylmorpholin. Vorzugsweise arbeitet man bei 10—30°, insbesondere bei Raumtemperatur.

Bei der Variante c) kann die erste Phase, d.h. die Bildung des Säureazids durch Behandlung mit salpetriger Säure oder einem Ester davon, mit Vorteil bei einer wesentlich höheren Konzentration der Ausgansstoffe als die nachfolgende Cyclisierung erfolgen. Zweckmässig arbeitet man mit etwa einem Aequivalent eines Niederalkylnitrits, wie Aethyl-, Isoamyl- und insbesondere tert-Butyl-nitrit, in salzsaurem Milieu bei Temperaturen von etwa —30° bis etwa —5°, vorzugsweise etwa —20°; ein geringer Ueberschuss an Nitrit ist zulässig. Danach wird die Lösung des bebildeten Azids nach der notwendigen Verdünnung bei einer Temperatur von etwa 0° bis etwa 35° mittels einer überschüssigen organischen Base, z.B. einer der oben genannten, basisch gestellt und dadurch wie bei der Verfahrensvariante b zur spontanen Cyclisierung gebracht.

Die linearen Peptide der Formel III, sowie die zu ihrer Synthese dienenden Zwischenprodukte, sind neu und zur Synthese auch anderer Somatostatin-Analogen, z.B. solcher mit längeren Aminosäure-Sequenzen, mit Vorteil anwendbar. Sie gehören, sowie auch ihre Herstellungsverfahren, zum Gegenstand der vorliegenden Erfindung. Sie werden nach an sich bekannten Methoden erhalten, indem man die zu ihrem Aufbau nötigen Aminosäuren bzw. kleinere Peptideinheiten unter Bildung von CONH-Bindungen in beliebiger zeitlicher Reihenfolge miteinander kondensiert, wobei nicht an der Reaktion teilnehmende funktionelle Gruppen intermediär geschützt werden können.

Bei der Herstellung dieser Ausgangstoffe, wie auch aller benötigten Zwischenprodukte, kommen als Schutzgruppen für die endständigen $\alpha$-Amino- und Carboxylgruppen besonders die bei der Synthese langkettiger Peptide üblichen Schutzgruppen in Betracht, die z.B. durch Solvolyse oder Reduktion leicht und selektiv abgespalten werden können.

Als $\alpha$-Amino-Schutzgruppen sind beispielsweise zu nennen:gegebenenfalls, z.B. durch Halogen, Nitro, Niederalkyl oder Niederalkoxy, substituierte Di- oder Triarylniederalkylgruppen, wie Diphenylmethyl oder Triphenylmethylgruppen, z.B. Benzhydryl, Trityl, Di-(p-methoxy)-benzhydryl, oder vor allem von der Kohlensäure sich ableitende hydrogenolytisch abspaltbare Gruppen, wie gegebenenfalls im aromatischen Rest durch Halogenatome, Nitrogruppen, Niederalkyl- oder Niederalkoxygruppen substituierte Benzyloxycarbonylgruppen, z.B. Benzyloxycarbonyl (d.h. Carbobenzoxy), p-Brom- oder p-Chlorbenzyloxycarbonyl, p-Nitrobenzyloxycarbonyl, p-Methoxybenzyloxycarbonyl; ferner auch 2-(p-Biphenylyl)-2-propyloxycarbonyl und ähnliche im Schweizer Patent 509 266 beschriebene Aryloxycarbonylgruppen. Dabei ist zu beachten, dass die $\alpha$-Aminoschutzgruppe selektiv unter Erhaltung der gegebenenfalls vorhandenen $\varepsilon$-Aminoschutzgruppe des Lysinrests abspaltbar sein muss. Es ist übrigens auch von Vorteil, wenn bei ihrer Abspaltung auch eine gegebenenfalls vorhandene Carboxyl- und Hydroxylschutzgruppe unversehrt bleibt.

Carboxylgruppen werden beispielsweise durch Hydrazidbildung oder durch Veresterung geschützt. Zur Veresterung geeignet sind z.B. niedere, gegebenenfalls substituierte Alkanole wie Methanol, Aethanol, Cyanmethylalkohol, 2,2,2-Trichloräthanol, Benzoylmethylalkohol oder insbedere tert-Butylalkohol, aber auch ein gegebenenfalls substituierter Benzylalkohol. Eine besonders vorteilhafte Kategorie der substituierten Alkanole sind Aethylalkohole, die in $\beta$-Stellung eine trisubstituierte Silylgruppe, wie Triphenylsilyl-, Dimethyl-butyl-silyl- oder vor allem Trimethylsilylgruppe, tragen. Wie z.B. im belgischen Patent Nr. 851.576 beschrieben ist, eignen sich diese Alkohole zum Schutze der Carboxylgruppen besonders gut deshalb, weil die entsprechenden $\beta$-Silyläthylester, z.B. $\beta$-(Trimethylsilyl)-äthylester, zwar die Stabilität üblicher Alkylester besitzen, jedoch sich unter minden Bedingungen durch Einwirkung von Fluoridionen unter Erhaltung aller anderen Schutzgruppen selektiv abspalten lassen.

Zur Bildung von aktivierten Estern, wie z.B. in den Verbindungen der Formel III, eignen sich z.B. gegebenenfalls durch elektronenanziehende Substituenten substituierte Phenole und Thiophenole wie Phenol, Thiophenol, Thiokresol, p-Nitrothiophenol, 2,4,5- und 2,4,6-Trichlorphenol, Pentachlorphenol, o- und p-Nitrophenol, 2,4-Dinitrophenol, p-Cyanophenol, weiter z.B. N-Hydroxysuccinimid, N-Hydroxyphthalimid und N-Hydroxypiperidin.

Die Hydroxylgruppe des Threoninrests kann durch Veresterung oder Verätherung, wie bereits oben angegeben, geschützt werden, sie kann jedoch auch frei bleiben.

Diese Schutzgruppen können in bekannter Weise abgespalten werden. So kann man die

Benzyloxycarbonylgruppe durch Hydrogenolyse, die N-Tritylgruppe mit Mineralsäuren, wie Halogenwasserstoffsäuren, z.B. Fluorwasserstoff oder vorzugsweise Chlorwasserstoff, oder einer organischen Säure, wie Ameisensäure, Essigsäure, Chloressigsäure oder Trifluoressigsäure, in wässerigem oder absolutem Trifluoräthanol als Lösungsmittel (vgl. deutsch Offenlegungsschrift DT 2 346 147) oder mit wässeriger Essigsäure, die tert-Butyloxycarbonylgruppe mit Trifluoressigsäure oder Salzsäure, die 2-(p-Biphenylyl)-isopropyloxycarbonylgruppe mit wässeriger Essigäure oder z.B. mit einem Gemisch von Eisessig, Ameisensäure (82,8%ig) und Wasser (7:1:2) oder nach dem Verfahren der DT 2 346 147 abspalten.

Die $\beta$-Silyläthylestergruppen werden vorzugsweise mit Fluoridionen-abgebenden Reagentien, z.B. Fluoriden quaternärer organischer Basen, wie Tetraäthylammoniumfluorid, abgespalten. Sie können aber auch, gleich wie die üblichen Alkylester, durch alkalische Hydrolyse, z.B. mittels Alkalimetallhydroxiden, -carbonaten oder -bicarbonaten, abgespalten werden oder durch Hydrazinolyse, z.B. mittels Hydrazinhydrat, in die entsprechenden Carbazoylgruppen umgewandelt werden. Zur Abspaltung von tert-Butylestern verwendet man vorzugsweise Acidolyse, für Benzylester dann Hydrogenolyse.

Die zur Herstellung der linearen Peptide der Formel III durchzuführende Kondensation der Aminosäure- und/oder Peptideinheiten erfolgt in an sich bekannter Weise, indem man vorzugsweise eine Aminosäure oder ein Peptid mit geschützter $\alpha$-Aminogruppe und gegebenenfalls aktivierter terminaler Carboxylgruppe (= aktive Komponente) an eine Aminosäure oder ein Peptid mit freier $\alpha$-Aminogruppe und freier oder geschützter, z.B. veresterter terminaler Carboxylgruppe (= passive Komponente), anknüpft, im gebildeten Produkt die terminale Aminogruppe freisetzt und dieses Peptid, enthaltend eine freie $\alpha$-Aminogruppe und eine ggf. geschützte terminale Carboxylgruppe, wieder mit einer weiteren aktiven Komponente, d.h. einer Aminosäure oder einem Peptid mit aktivierter terminaler Carboxylgruppe und freier $\alpha$-Aminogruppe, umsetzt, usw. Die Carboxylgruppe kann beispielsweise durch Ueberführung in ein Säureazid, -anhydrid, -imidazolid, isoxazolid oder einen aktivierten Ester, wie einen der oben genannten, oder durch Reaktion mit einem Carbodiimid, wie N,N'-Dicyclohexylcarbodiimid, gegebenenfalls unter Zusatz von N-Hydroxysuccinimid oder einem unsubstituierten oder z.B. durch Halogen, Methyl oder Methoxy substituierten 1-Hydroxybenzotriazol oder 4-Hydroxy-benzo-1,2,3-triazin-3-oxid (u.a., vgl. DE—A—1 917 690, DE—A—1 937 656, DE—A—2 202 613) oder mit N,N'-Carbonyldiimidazol aktiviert werden. Als die gebräuchlichste Kupplungsmethode ist die Carbodiimidmethode zu nennen, ferner auch die Azidmethode, die Methode der aktivierten Ester und die Anhydridmethode, sowie die Merrifield-Methode und die Methode der N-Carboxyanhydride oder N-Thiocarboxyanhydride.

Bei einer besonders bevorzugten Herstellung der linearen Peptide der Formel II verwendet man als die Kupplungsmethode die Carbodiimid-Methode mit N,N'-Dicyclohexylcarbodiimid in Anwesenheit von 1-Hydroxybenzotriazol. Die terminale Carboxylgruppe wird dabei in Form des $\beta$-(Trimethyl-silyl)-äthylesters geschützt, die $\alpha$-Aminogruppe der aktiven Komponente wird durch die Benzyloxycarbonylgruppe geschützt, die nach jedem Kupplungsschritt durch Hydrogenolyse abgespalten wird. Zum Schutz der $\varepsilon$-Aminogruppe des Lysinrest wird die Acylierung mit der tert-Butoxycarbonylgruppe und für die Hydroxylgruppe des Threoninrests die Verätherung mit der tert-Butylgruppe verwendet. Diese beiden Schutzgruppen können, wenn erwünscht, zuletzt in einem Schritt durch saure Hydrolyse, z.B. mittels Trifluoressigsäure, Salzsäure oder Fluorwasserstoff, abgespalten werden.

Je nach Arbeitsweise erhält man die Verbindungen in Form von Basen oder ihren Salzen. Aus den Salzen können in an sich bekannter Weise die Basen gewonnen werden. Von letzteren wiederum lassen sich durch Umsetzen mit Säuren, z.B. mit solchen, die die oben genannten Salze bilden, therapeutisch anwendbare Säureadditionssalze gewinnen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahren, bei denen man von einer auf beliebiger Verfahrensstuffe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Die vorliegende Erfindung betrifft ebenfalls pharmazeutische Präparate, welche Verbindungen der Formel I oder pharmazeutisch verwendbare Salze davon enthalten. Diese pharmazeutischen Präparate können insbesondere in den oben angegebenen Indikationen Verwendung finden, wenn sie intraperitoneal, wie intravenös, intramuskulär oder subcutan, oder auch intranasal verabreicht werden. Die erforderliche Dosis hängt von der speziellen zu behandelnden Erkrankung, ihrer Schwere und von der Dauer der Therapie ab. Anzahl und Menge der Einzeldosen, sowie das Verabreichungsschema kann am besten anhand einer individuellen Untersuchung des jeweiligen Patienten bestimmt werden. Die Methode zur Bestimmung dieser Faktoren ist dem Fachmann geläufig. In der Regel liegt jedoch bei einer Injektion eine therapeutisch wirksame Menge einer solchen Verbindung im Dosisbereich von etwa 0,001 bis etwa 0,2 mg/kg Körpergewicht vor. Bevorzugt wird der Bereich von etwa 0,0015 bis etwa

0,15 mg/kg Körpergewicht und die Verabreichung erfolgt durch intravenöse Infusion oder subkutane Injektion. Dementsprechend enthalten pharmazeütische Präparate zur parenteralen Verabreichung in Einzeldosis-Form in Abhängigkeit von der Applikationsart pro Dosis etwa 0,08 bis etwa 15 mg einer der erfindungsgemässen Verbindungen. Neben dem Wirkstoff enthalten sie üblicherweise noch einen Puffer, z.B. Ein Phosphatpuffer, der das pH zwischen etwa 3,5 und 7 halten soll, ferner Natriumchlorid, Mannit oder Sorbit zur Einstellung der Isotonie. Sie Können in gefriergetrockneter oder gelöster Form vorliegen, wobei Lösungen mit Vorteil ein Antibakteriell wirkendes Konservierungsmittel, z.B. 0.2—0,3% 4-Hydroxybenzoesäure-methylester oder -äthylester enthalten können. Soll in solchen Präparaten der Wirkstoff in Form eines Komplexes mit verlängerter Wirkungsdauer vorliegen, so kann dieser direkt durch Zusatz der komplexbildenden Komponenten zu einer Injektionslösung, die z.B. gemäss der oben erwähnten Massnahmen zubereitet werden, gebildet werden. Als Zusatz eignet sich z.B. 0,1—1,0 Gewichts-% eines Zink(II)-Salzes (z.B. Sulfats) in Verbindung mit 0,5—5,0 Gewichts-% Protamin (z.B. als Sulfat), bezogen auf das Gesamtvolumen der Injektionslösung; diese Zubereitung liegt als Lösung von pH 3,5 bis etwa 6,5 oder als Suspension von etwa pH 7,5 bis 8,0 vor.

Ein Präparat für die intranasale Verabreichung kann als eine wässrige Lösung oder Gelee, eine ölige Lösung oder Suspension, oder aber eine fetthaltige Salbe vorliegen. Ein Präparat in Form einer wässrigen Lösung erhält man z.B. dadurch, dass man den Wirkstoff der Formel I, oder ein therapeutisch anwendbares Säureadditionssalz davon in einer wässrigen Pufferlösung von pH bis 7,2 löst und einen Isotonie-erzeugenden Stoff zusetzt. Der wässrigen Lösung wird zweckmässig ein polymeres Haftmittel, z.B. Polyvinylpyrrolidon, und/oder ein Konservierungsmittel zugefügt. Die Einzeldosis beträgt etwa 0,08 bis etwa 15 mg, vorzugsweise 0,25 bis 10 mg, die in etwa 0,05 ml einer Lösung bzw. 0,05 g eines Gelees enthalten sind.

Eine ölige Applikationsform für die intranasale Verabreichung erhält man z.B. durch Suspendieren eines Peptids der Formel I oder eines therapeutisch anwendbaren Säureadditionssalzes davon in einem Oel, gegebenenfalls unter Zusatz von Quellmitteln, wie Aluminiumstearat, und/oder grenzflächenaktiven Mitteln (Tensiden), deren HLB-Wert ("hydrophilic-lipophilic-ballance") unter 10 liegt, wie Fettsäuremonoester mehrwertiger Alkohole, z.B. Glycerinmonostearat, Sorbitanmonolaurat, Sorbitanmonostearat oder Sorbitanmonoleat.—Eine fetthaltige Salbe erhält man z.B. durch Suspendieren des erfindungsgemässen Wirkstoffes in einer streichbaren Fettgrundlage, gegebenenfalls unter Zusatz eines Tensids vom HLB-Wert unter 10. Eine Emulsionssalbe erhält man durch Verreiben einer wässrigen Lösung des peptidischen Wirkstoffes in einer weichen, streichbaren Fettunterlage unter Zusatz eines Tensids, dessen HLB-Wert unter 10 liegt. Alle diese intranasale Applikationsformen können auch Konservierungsmittel enthalten.—Die Einzeldosen betragen etwa 0,08 bis etwa 15 mg, vorzugsweise 0,25 bis 10 mg, enthalten in etwa 0,05 bis etwa 0,1 g der Grundmasse.

Für die intranasale Verabreichung eignen sich weiter auch Inhalations- bzw. Insufflationspräparate, wie Insufflationskapseln, die den Wirkstoff in Form eines Puders mit der Atemluft zu insufflieren gestatten, oder Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise Hilfsmittel; Insufflationskapseln enthalten z.B. feste Trägerstoffe, wie Lactose; Aerosol-bzw. Sprayzubereitungen enthalten z.B. ein flüssiges Treibgas mit einem Siedepunkt unter der Raumtemperatur, sowie, wenn erwünscht, weiter Trägerstoffe, wie flüssige oder feste nicht-ionische oder anionische Tenside und/oder feste Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, die mittels einer geeigneten Verdichtungs- und Entspannungsvorrichtung nach Bedarf erzeugt wird.

Die Erfindung betrifft ebenfalls die Verwendung der neuen Verbindungen der Formel I und therapeutisch anwendbaren Säureadditionssalzen davon als pharmakologisch aktive Verbindungen, insbesondere in den für Somatostatin üblichen Indikationen, vorzugsweise in der Form von pharmazeutischen Präparaten. Die tägliche Dosis, die einem Warmblüter von etwa 70 kg verabreicht wird, beträgt von etwa 0,1 bis etwa 120 mg.

In den nachfolgenden Beispielen wird die Erfindung illustriert, jedoch durch diese nicht eingeschränkt. Temperaturen werden in Celsiusgraden angegeben; als Abkürzungen, z.B. zur Bezeichnung von Aminosäuren, Peptiden, Schutzgruppen, etc., werden die üblichen, z.B. die in "Synthese von Peptiden" (Herausgeber: E. Wünsch), Bd XV der "Methoden der org. Chemie" (Houben-Weyl) (1974; G. Thieme, Stuttgart), zusammengestellten Kurzbezeichnungen verwendet. Insbesondere werden folgende Abkürzungen verwendet.

| | | |
|---|---|---|
| Boc | — | tert-Butoxycarbonyl |
| Bpoc | — | 2-(p-Biphenylyl)-2-propyloxycarbonyl |
| But | — | tert-Butyl (als ätherbildende Gruppe) |
| DCCI | — | N,N'-Dicyclohexylcarbodiimid |

Gaba — 4-Aminobuttersäure—Rest, —NH—$(CH_2)_3$—CO—

OBzl — Benzyloxy (als esterbildende Gruppe)

ONP — p-Nitrophenoxy (als esterbildende Gruppe)

ONSu — Succinimido-N-oxy

OTmse — 2-(Trimethylsilyl)-äthoxy (als esterbildende Gruppe)

SEOC — 2-(Trimethylsilyl)-äthoxycarbonyl

Z — Benzyloxycarbonyl (Carbobenzoxy)

DC — Dünnschichtchromatographie

In DC verwendet man, wenn nichts anderes angegeben ist, Kieselgel als Adsorbens und folgende Systeme als Laufmittel:

System 45 : sek-Butanol-3%-ige wässriges Ammoniak (7:3)

System 52 : n-Butanol-Essigsäure-Wasser (71,5:7,5:21)

System 101 : n-Butanol-Pyridin-Essigsäure-Wasser (38:24:8:30)

System 101B : n-Butanol-Pyridin-25%-iges wässeriges Ammoniak-Wasser (40:24:6:30)

System 112A : n-Butanol-Pyridin-Ameisensäure-Wasser (42:24:4:20).

System 157 : Chloroform-Methanol-Essigsäure-Wasser (70:42:0,5:10)

System 157A : Chloroform-Methanol-Essigsäure-Wasser (90:10:0,5:1)

System 157B : Chloroform-Methanol-Essigsäure-Wasser (85:13:0,5:1,5)

System 157C : Chloroform-Methanol-Essigsäure-Wasser (75:26:0,5:5)

Beispiel 1

Asn-Phe-Phe-(D-trp)-Lys-Thr-Phe-Gaba (cyclisch)

195 mg geschütztes Oktapeptid der Formel Asn-Phe-Phe-(D-trp)-Lys(Boc)-Thr(But)-Phe-Gaba (cyclisch) wird bei 5° unter $N_2$ in 1,5 ml eines Gemisches aus 89 Vol.% Trifluoressigsäure, 10 Vol.% Wasser und 1 Vol.% Thioglykolsäure gelöst, die Lösung sofort auf 25° erwärmt und nach 90 Minuten bei Raumtemperatur unter $N_2$ mit 15 ml Aether ausgefällt. Das resultierende rohe Trifluoracetat des Endprodukts wird im Vakuum getrocknet, in 5 ml 1 N Essigsäure gelöst und durch 15 ml Anionenaustauscher (z.B. AG® 1—X8, ein Produkt von Bio-Rad Laboratories, Richmond, California, USA), in Acetatform, filtriert. Das Eluat wird im Vakuum eingedampft und der Rückstand der Gegenstromverteilung im System n-Butanol-Essigsäure-Wasser (2400:600:3000) über 200 Stufen unterzogen. Die in den Elementen 168 bis 183 enthaltenen Phasen (K = 5.9) werden gesammelt, im Vakuum eingedampft und aus tert-Butanol-Wasser (1:1) lyophilisiert.

Die erhaltene Titelverbindung ist in drei Systemen dünnschichtenchromatographisch einheitlich.
DC (Cellulose, Merck): System 101 : $R_f$ 0.9
111B: $R_f$ 0.9
112A: $R_f$ 0.8

Der peptidische Ausgangsstoff ist folgendermassen erhältlich:

*Stufe 1.1*
H-Phe-OTmse-Hydrochlorid

Eine Lösung von 2,50 g Z-Phe-OTmse in 25 ml Methanol wird nach Zugabe von 0,25 g Palladium-Kohle (10%) bei Raumtemperatur und Normaldruck während 3 Stunden hydriert, wobei das pH des Reaktionsgemisches durch Zugabe von 1N methanolischer Chlorwasserstofflösung auf 5.5 gehalten wird. Zur Aufarbeitung wird der Katalysator abfiltriert und das Filtrat im Vakuum eingedampft. Das Rohprodukt kann ohne weitere Reinigung in der nächsten Stufe eingesetzt werden.

*Stufe 1.2*

Z-Thr(But)-Phe-OTmse

Einer Lösung von 10,30 g Z-Thr(But)OH in 120 ml wasserfreiem Tetrahydrofuran und 4,20 ml N-Aethylmorpholin wird während 3 Minuten bei —15° eine Lösung von 4,43 ml Chlorameisensäureisobutylester in 20 ml Tetrahydrofuran zugefügt. Nach 10 Minuten bei —10° bis —15° wird bei —15° eine Lösung von 10,05 g H-Phe-OTmse-Hydrochlorid (s. Stufe 1.1) in 40 ml Tetrahydrofuran und eine weitere Portion von 4,20 ml N-Aethylmorpholin zugefügt und das Reaktionsgemisch während je 1 Stunde bei —15°, bei 0° und bei Raumtemperatur gehalten. Zur Aufarbeitung wird das ausgefallene N-Aethylmorpholinhydrochlorid abfiltriert, das Filtrat im Vakuum eingedampft, der Rückstand in Aethylacetat gelöst und je dreimal mit 1N-Zitronensäure, 1N-Natriumhydrocarbonat und Wasser gewaschen. Die organische Phase wird über Natriumsulfat getrocknet, im Vakuum eingedampft und im Hochvakuum von letzten Spuren der Lösungsmittel befreit. Das Produkt wird als ein dickflüssiges, gelbliches Oel erhalten, das gemäss DC einheitlich ist.

DC: [Chloroform-Methanol (9:1)] $R_f$ 0,83
[Aethylacetat-Petroläther (7:3)] $R_f$ 0,77

*Stufe 1.3*

H-Thr(But)-Phe-OTmse

Eine Lösung von 8,84 g Z-Thr(But)-Phe-OTmse (Stufe 1.2) in 325 ml Methanol wird nach Zugabe von 880 mg Palladium-Kohle (10%) während 5 Stunden bei Normaldruck und Raumtemperatur hydriert. Zur Aufarbeitung wird der Katalysator abfiltriert und das Filtrat im Vakuum eingedampft. Der ölige Rückstand des Produktes ist gemäss DC einheitlich und wird ohne Reinigung in Stufe 1.4 eingesetzt.

DC: [Chloroform-Methanol (9:1)] $R_f$ 0,62

*Stufe 1.3A*

Z-(D-trp)-Lys(Boc)-OH

Zu einer Lösung von 33,84 g Z-(D-trp)-OH und 17,42 g 8-Hydroxy-chinolin in 50 ml Acetonitril wird bei 0°—5° eine Lösung von 21,87 g DCCI in 100 ml Acetonitril während 45 Minuten zugetropft. Nach weiteren 30 Minuten bei 5° wird der ausgefallene Dicyclohexylharnstoff durch Filtration und Auswaschen mit 50 ml Acetonitril entfernt. Das Filtrat versetzt man mit einer Lösung von 27,09 g H-Lys(Boc)-OH in 25,9 ml 4,25N-Kalilauge und 80 ml Acetonitril und lässt während 15 Stunden bei Raumtemperatur stehen. Zur Aufarbeitung wird das Reaktionsgemisch im Vakuum eingedampft, der Rückstand in 1 Liter Aethylacetat aufgenommen, dreimal mit je 200 ml 1N-Salzsäure bei 0° und dreimal mit je 200 ml Wasser gewaschen, die organische Phase über Natriumsulfat getrocknet und im Vakuum eingedampft. Das erhaltene braune Oel wird in 150 ml Chloroform gelöst und unter starkem Rühren in 1,5 Liter Hexan getropft. Die flokkige, klebrige Ausfällung wird abfiltriert, mit 500 ml Hexan gewaschen und im Vakuum getrocknet. Zur weiteren Reinigung wird das Material in 150 ml Tetrachlorkohlenstoff-Aethylacetat (6:4 Vol.-Teile) gelöst und auf einer Kieselgelsäule mit diesem Lösungsmittel-Gemisch chromatographiert. Geeignete dünnschichtenchromatographisch einheitliche Fraktionen werden im Vakuum eingedampft, wodurch das reine Produkt in Form einer schaumartigen Mass erhalten wird.

DC: [Chloroform-Methanol-Wasser (14:6:1)] $R_f$ 0,61

*Stufe 1.4*

Z-(D-trp)-Lys(Boc)-Thr(But)-Phe-OTmse

Eine Lösung von 7,24 g rohem (87%-ig gemäss Titrierung) H-Thr(But)-Phe-OTmse (Stufe 1.3) und 8,45 g Z-(D-trp)-Lys(Boc)-OH (Stufe 1.3A) in 100 ml Dimethylformamid wird bei 5° mit 2,28 g N-Hydroxy-benzotriazol und 3,38 g DCCI versetzt und das Reaktionsgemisch während 1 Stunde bei 5° und weitere 15 Stunden bei Raumtemperatur gehalten. Zur Aufarbeitung wird der ausgefallene Dicyclohexylharnstoff durch Abfiltrieren entfernt und das Filtrat im Hochvakuum eingedampft. Der Rückstand wird zweimal aus Aethylacetat-Petroläther umkristallisiert und im Vakuum getrocknet, Smp. 114—120°.

DC: [Chloroform-Aethylacetat (1:1)] $R_f$ 0,23;
[Toluol-Aceton (1:1)] $R_f$ 0,70

*Stufe 1.5*

H-(D-trp)-Lys(Boc)-Thr(But)-Phe-OTmse

Eine Lösung von 5,00 g Z-(D-trp)-Lys(Boc)-Thr(But)-Phe-OTmse (Stufe 1.4) in 300 ml Methanol wird nach Zugabe von 0,50 g Palladium-Kohle (10%) bei Raumtemperatur und Normaldruck während 5 Stunden hydriert. Zur Aufarbeitung wird vom Katalysator abfiltriert und der nach dem Eindampfen der Filtrates verbleibende Rückstand sofort in Stufe 1.6 weiter umgesetzt.

*Stufe 1.5A*

Z-Asn-Phe-Phe-OH

Zu einer Suspension von 2,95 g H-Phe-Phe-OH in 30 ml Dimethylformamid und 6 ml Wasser wird 2,36 ml 4N-Natronlauge und nachher 5,29 g Z-Asn-ONP zugefügt und während 20 Stunden bei 35° gut gerührt. Zur Aufarbeitung wird die Reaktionslösung bei 5° mit 2,36 ml 4N-Salzsäure versetzt, die schwach getrübte Lösung filtriert und das Filtrat im Hochvakuum auf ca. 15 ml konzentriert. Durch Zugabe von 150 ml Wasser wird das Rohprodukt in gallertartiger Form gefällt, abfiltriert und im Vakumm über Phosphorpentoxid getrocknet. Dieses Material wird noch zweimal aus je 20 ml Methanol und 50 ml Aether umgefällt, abfiltriert und im Vakuum getrocknet. Gemäss dem Racemisierungstest (Totalhydrolyse, Derivatisierung der Aminosäuren und Auftrennung durch Gaschromatographie) liegen in diesem Produkt weniger als 2% der Aminosäuren in der D-Konfiguration vor.

DC: [Chloroform-Methanol-Essigsäure-Wasser (70:40:0,5:10)] $R_f$ 0,50

*Stufe 1.6*

Z-Asn-Phe-Phe-(D-trp)-Lys(Boc)-Thr(But)-Phe-OTmse

Eine Lösung von 632 mg Z-Asn-Phe-Phe-OH (Stufe 1.5A) und 862 mg H-(D-trp)-Lys(Boc)-Thr(But)-Phe-OTmse (Stufe 1.5) in 5 ml Dimethylformamid wird mit 210 mg N-Hydroxy-benzotriazol und 276 mg DCCI versetzt und 15 Stunden bei Raumtemperatur belassen. Zur Aufarbeitung wird der ausgefallene Dicyclohexylharnstoff durch Abfiltrieren beseitigt und das Filtrat im Hochvakuum eingedampft. Der ölige Rückstand wird mit 5 ml Methanol verrieben und abgesaugt. Das ungelöste Material wird zur Reinigung nochmals mit 5 ml Methanol bei 50° verrieben, abgesaugt, mit Methanol gewaschen und im Vakuum getrocknet. Das Produkt ist gemäss DC einheitlich.

DC: [Chloroform-Methanol (85:15)] $R_f$ 0,85
[Chloroform-Methanol-Wasser (14:6:1)] $R_f$ 0,90

*Stufe 1.7*

Z-Asn-Phe-Phe-(D-trp)-Lys(Boc)-Thr(But)-Phe-OH

Z-Asn-Phe-Phe-(D-trp)-Lys(Boc)-Thr(But)-Phe-OTmse (Stufe 1.6) (940 mg) wird in 23 ml einer frisch hergestellten, wasserfreien 0,15 M-Tetraäthylammoniumfluorid-Lösung in Dimethylformamid gelöst und 30 Minuten bei 25° gehalten. Nach dem Abkühlen auf 5° wird das Reaktionsgemisch unter gutem Umrühren mit 0,68 ml 1 N wässeriger Salzsäure versetzt und das Produkt durch Zugabe von 70 ml Wasser gefällt. Das abfiltrierte Material wird mit 5 ml Wasser gewaschen, im Vakuum über Phosphorpentoxid getrocknet und direkt in Stufe 1.8 eingesetzt.

*Stufe 1.7A*

H-Gaba-OBzl-p-toluolsulfonat

Ein Gemisch von 30,94 g 4-Aminobuttersäure und 68,48 g p-Toluolsulfonsäure-monohydrat in 311 ml Benzylalkohol und 300 ml Benzol wird bei Normaldruck langsam destilliert, bis während 5 Stunden insgesamt 200 ml einer Fraktion, Sdp. 70°—90° aufgefangen wird. Die klare Reaktionslösung wird im Wasserstrahlvakuum und dann im Hockvakuum bei ca. 60° auf 150 ml konzentriert. Der ausgefallene Kristallbrei wird mit Aether verrührt, die Kristalle abfiltriert und mit 200 ml Aether gewaschen. Zur weiteren Reinigung wird dieses Material mit 500 ml Aether bei Raumtemperatur während einer Stunde verrührt, abfiltriert, mit Aether gewaschen und im Vakuum getrocknet, Smp. 106—107,5°.

DC: [Chloroform-Methanol-17%-iges wässeriges Ammoniak-Wasser (50:40:6:4)] $R_f$ 0,39

*Stufe 1.8*

Z-Asn-Phe-Phe-(D-trp)-Lys(Boc)-Thr(But)-Phe-Gaba-OBzl

Ein Gemisch von 430 mg Z-Asn-Phe-Phe-(D-trp)-Lys(Boc)-Thr(But)-Phe-OH (Stufe 1.7) und 82 mg Gaba-benzylester (freigesetzt durch Zugabe von 0,046 ml N-Methylmorpholin zu 152 mg entsprechendem p-Toluolsulfonat, siehe Stufe 1.7A) in 2 ml Dimethylformamid wird mit 61 mg N-Hydroxybenzotriazol und 93 mg DCCI versetzt und 20 Stunden bei Raumtemperatur belassen. Zur Aufarbeitung wird das Gemisch mit 10 ml eiskaltem Methanol versetzt und abfiltriert Der gewonnene Feststoff wird zur weiteren Reinigung mit 5 ml warmem Methanol während 10 Minuten verrührt, die Suspension auf 0° abgekühlt, das reine Produkt abfiltriert und im Vakuum getrocknet.

DC: [Chloroform-Methanol (85:15)] $R_f$ 0,85

*Stufe 1.9*

H-Asn-Phe-Phe-(D-trp)-Lys(Boc)-Thr(But)-Phe-Gaba-OH

Eine Lösung von 380 mg Z-Asn-Phe-Phe-(D-trp)-Lys(Boc)-Thr(But)-Phe-Gaba-OBzl (Stufe 1.8) in 25 ml Dimethylformamid wird nach Zugabe von 50 mg Palladium-Kohle (10%) während 6 Stunden bei Raumtemperatur und Normaldruck hydriert. Zur Aufarbeitung wird die Lösung nach Abfiltrieren des Katalysators im Hochvakuum auf 2 ml konzentriert und das Produkt mit 25 ml peroxidfreiem Aether ausgefällt, abfiltriert und im Vakuum getrocknet. Das Rohprodukt wird ohne weitere Reinigung der nächsten Stufe 1.10 (Cyclisierung) unterzogen.

*Stufe 1.10*

⌐Asn-Phe-Phe-(D-trp)-Lys(Boc)-Thr(But)-Phe-Gaba⌐

Eine Lösung von 297 mg rohem H-Asn-Phe-Phe-(D-trp)-Lys(Boc)-Thr(But)-Phe-Gaba-OH (Stufe 1.9) 324 mg N-Hydroxybenzotriazol und 495 mg DCCI in 240 ml Dimethylformamid wird während 20 Stunden bei 50° gehalten. Zur Aufarbeitung wird das Lösungsmittel im Hochvakuum bei ca. 30° abgedampft und der Rückstand mit 10 ml Aethylacetat verrieben. Der ausgefallene Dicyclohexylharnstoff wird durch Abfiltrieren beseitigt, das Filtrat mit Aethylacetat auf 50 ml verdünnt, dreimal mit je 20 ml 1N wässeriger Oxalsäure und dann mit Wasser bis zur Neutralität gewaschen, über Natriumsulfat getrocknet und im Vakuum eingedampft. Das Rohprodukt wird zur Reinigung der Gegenstromverteilung im System Methanol - Wasser - Chloroform - Tetrachlorkohlenstoff (2700:675:900:1575 Vol.-Teile) über 460 Stufen unterzogen. Die in den Elementen 198 bis 240 enthaltenen Phasen (K = 0,88) werden vereinigt und im Vakuum eingedampft. Der Rückstand wird in 20 ml tert-Butanol gelöst und lyophilisiert, womit ein dünnschichtenchromatographisch einheitliches Material der obigen Formel resultiert.

DC: [Chloroform-Methanol (85:15)] $R_f$ 0,18

[Chloroform-Methanol-Wasser (14:6:1)] $R_f$ 0,77

Beispiel 2

⌐Asn-Phe-Phe-(D-trp)-Lys-Thr-Phe-Gaba-Gaba⌐

In analoger Weise wie im Beispiel 1 beschrieben behandelt man das geschützte Nonapeptid der Formel ⌐Asn-Phe-Phe-(D-trp)-Lys(Boc)-Thr(But)-Phe-Gaba-Gaba⌐ mit Trifluoressigsäure und arbeitet auf.

Durch die Gegenstromverteilung (K = 5,45) an 200 Elementen wird das gewünschte Produkt als dünnschichtchromatographisch einheitliche amorphe Substanz erhalten.

DC: Cellulose, Merck;

System 101 : $R_f$ 0,90

111B: $R_f$ 0,75

112A: $R_f$ 0,93

Das als Ausgangsstoff verwendete geschützte Nonapeptid ist folgendermassen erhältlich:

*Stufe 2.1*

Boc-Gaba-OH

Eine Lösung von 41,25 g H-Gaba-OH in 320 ml Dioxan-Wasser (1:1 Vol. -Teile) wird mittels 20,5 ml 4N-Natronlauge auf pH 10,0 gestellt und mit 63,0 g Boc-azid versetzt. Durch langsame Zugabe von 4N-Natronlauge (insgesamt 185 ml) während 24 Stunden bei Raumtemperatur wird das pH auf 10,0 gehalten. Zur Aufarbeitung wird die Reaktionslösung dreimal mit je 250 ml Aether gewaschen und die wässerige Phase bei 5° durch Zugabe von 192 g Zitronensäure auf pH 3,0 gestellt. Das ausgefallene Material wird in drei Portionen 400 ml Aether aufgenommen und die vereinigten organischen Phasen neutral gewaschen, über Natriumsulfat getrocknet und im Vakuum eingedampft. Der Rückstand wird in 50 ml Aether gelöst und durch Zugabe von 100 ml Petrol-äther kristallisiert, Smp. 60°—62°.

DC: [Chloroform-Methanol-Wasser (14:6:1] $R_f$ 0,83

[Benzol-Aceton (7:3)] $R_f$ 0,21

*Stufe 2.2*

Boc-Gaba-Gaba-OBzl

Eine Lösung von 2,03 g Boc-Gaba-OH (Stufe 2.1) in 20 ml wasserfreiem Tetrahydrofuran wird bei −15° zuerst mit 1,39 ml Triäthylamin und dann während 1 Minute mit 1,31 ml Chlorameisensäure-isobutylester versetzt. Nach 10 Minuten bei −10° bis −15° wird bei −15° eine Lösung von 3,65 g H-Gaba-OBzl-p-toluolsulfonat (Beispiel 1.7A) und 1,39 ml Triäthylamin in 10 ml Dimethylformamid zugegeben und das Reaktionsgemisch während je 1 Stunde bei −10°, 0° und Raumtemperatur gehalten. Zur Aufarbeitung wird das Gemisch mit 150 ml Aethylacetat versetzt, und je dreimal mit 1N-Zitronensäure, 1N-$NaHCO_3$ und mit Wasser gewaschen. Die organische Phase wird nach dem Trocknen über Natriumsulfat im Vakuum eingedampft und das verbleibende, schwach gelblich gefärbte Oel im Hochvakuum von Resten der Lösungsmittel befreit. Das Produkt ist gemäss DC einheitlich.

DC [Chloroform-Methanol (85:15)] $R_f$ 0,60

[Toluol-Aceton (7:3)] $R_f$ 0,17

*Stufe 2.3*

H-Gaba-Gabe-OBzl-Hydrochlorid

2,00 g Boc-Gaba-Gaba-OBzl (Stufe 2.2) wird in 15 ml einer 2N-Lösung von Chlorwasserstoff in Aethylacetat gelöst und 1 Stunde bei Raumtemperatur belassen. Nach dem Abkühlen des

Reaktionsgemisches auf 0° wird das ausgefallene Produkt abfiltriert, mit 20 ml Aether gewaschen und im Vakuum getrocknet, Smp. 80—83°.
DC: [Chloroform-Methanol-Wasser (14:6:1)] $R_f$ 0,29

*Stufe 2.4*
Z-Asn-Phe-Phe-(D-trp)-Lys(Boc)-Thr(But)-Phe-Gaba-Gaba-OBzl

Zu einer Lösung von 430 mg Z-Asn-Phe-Phe-(D-trp)-Lys(Boc)-Thr(But)-Phe-OH (Beispiel 1.7) und 142 mg H-Gaba-Gaba-OBzl-Hydrochlorid (Stufe 2.3) in 3 ml Dimethylformamid wird 45 mg Triäthylamin, 61 mg 1-Hydroxy-benzotriazol sowie 93 mg N,N'-Dicyclohexylcarbodiimid zugefügt und 24 Stunden bei Raumtemperatur stehen gelassen. Das Reaktionsgemisch wird mit 15 ml Wasser versetzt und das ausgefallene Material abfiltriert und im Vakuum über Phosphorpentoxid getrocknet. Zur Reinigung wird das Rohprodukt mit 5 ml heissem Methanol verrieben, abgesaugt und nach dem Trocknen im Vakuum aus einer Lösung in 2 ml Dimethylformamid mit 10 ml Methanol gefällt. Die Fällung wird abfiltriert, mit wenig Methanol gewaschen und im Vakuum getrocknet, wodurch ein dünnschichtchromatographisch einheitliches Produkt resultiert.
DC: [Chloroform-Methanol (85:15)] $R_f$ 0,80

*Stufe 2.5*
H-Asn-Phe-Phe-(D-trp)-Lys(Boc)-Thr(But)-Phe-Gaba-Gaba-OH

Diese Verbindung wird in analoger Weise wie im Beispiel 1.9, durch Hydrierung von Z-Asn-Phe-Phe-(D-trp)-Lys(Boc)-Thr(But)-Phe-Gaba-Gaba-OBzl (Stufe 2.4) erhalten und ohne Reinigung in Stufe 2.6 weiterverarbeitet.

*Stufe 2.6*
cyclo[Asn-Phe-Phe-(D-trp)-Lys(Boc)-Thr(But)-Phe-Gaba-Gaba]

Diese Verbindung wird in analoger Weise wie im Beispiel 1.10, durch Cyclisierung von H-Asn-Phe-Phe-(D-trp)-Lys(Boc)-Thr(But)-Phe-Gaba-Gaba-OH (Stufe 2.5) mittels DCCI und 1-Hydroxybenzotriazol erhalten. Das Rohprodukt wird durch Gegenstromverteilung (K=1,1) gereinigt.
DC: [Chloroform-Methanol (85:15)] $R_f$ 0,06
[Chloroform-Methanol-Wasser (14:6:1)] $R_f$ 0,69.

Beispiel 3

cyclo[Asn-Phe-(D-Trp)-Lys-Thr-Phe]

In analoger Weise, wie im Beispiel 1 beschrieben, wird das geschützte Peptid der Formel

cyclo[Asn-Phe-Phe-(D-Trp)-Lys(Boc)-Thr(But)-Phe]

mit Trifluoressigsäure behandelt und weiterverarbeitet. Durch Gegenstromverteilung (K=3,4) über 220 Stufen wird das gewünschte Produkt als amorphe Substanz erhalten
DC: System 101 $R_f$ 0,60
111B $R_f$ 0,37
112A $R_f$ 0,50

Das als Ausgangsstoff verwendete geschützte Peptid der oben angegebenen Formel ist folgendermassen erhältlich: 1,10 g Z-Asn-Phe-(D-Trp)-Lys(Boc)-Thr(But)-Phe-OH (Beispiel 1.7) werden in 30 ml Dimethylformamid unter Zusatz von 100 mg Pd-Kohle (10%) bis zur vollständigen Abspaltung der Z-Gruppe hydriert (ca. 4 h, DC-Kontrolle). Nach dem Abfiltrieren des Katalysators wird das Filtrat im Hochvakuum auf 15 ml konzentriert und die erhaltene Lösung des Peptids der Formel

H-Asn-Phe-Phe-(D-Trp)-Lys(Boc)-Thr(But)-Phe-OH

direkt in analoger Weise wie im Beispiel 1.10 mittels DCCI und N-Hydroxybenzotriazol cyclisiert. Das Rohprodukt wird durch Gegenstromverteilung (K=0,71) gereinigt, womit das gewünschte geschützte Cyclopeptid der Formel

cyclo[Asn-Phe-Phe-(D-Trp)-Lys(Boc)-Thr(But)-Phe] resultiert.
DC: [Chloroform-Methanol-Wasser (14:6:1)] $R_f$ 0.83

Beispiel 4

cyclo[Asn-Phe-Phe-(D-Trp)-Lys-Thr-Phe-NH$(CH_2)_4$CO]

In analoger Weise, wie im Beispiel 1 beschrieben, behandelt man das geschützte Peptid der

Formel └Asn-Phe-Phe-(D-Trp)-Lys(Boc)-Thr(But)-Phe-NH—$(CH_2)_4$—CO┘ mit Trifluoressigsäure und arbeitet auf. Durch Gegenstromverteilung (K=3,31) über 220 Stufen wird das gewünschte Produkt als amorphe Substanz erhalten.

DC: System 101 $R_f$ 0,29
111B $R_f$ 0,34
112A $R_f$ 0,47

Das als Ausgangsstoff verwendete geschützte Peptid wird folgendermassen erhalten:

*Stufe 4.1*

H-Asn-Phe-Phe-(D-Trp)-Lys(Boc)-Thr(But)-Phe-OTmse

942 mg Z-Asn-Phe-Phe-(D-Trp)-Lys(Boc)-Thr(But)-Phe-OTmse (Beispiel 1.6) werden in 50 ml Dimethylformamid unter Zusatz von 100 mg Pd-Kohle (10%) hydriert bis die Z-Gruppe vollständig abgespalten ist (ca 2 Stunden, DC-Kontrolle). Nach dem Abfiltrieren des Katalysators wird das Filtrat im Hochvakuum auf 4 ml eingeengt und direkt in der nächsten Stufe eingesetzt.

*Stufe 4.2*

Z--NH—$(CH_2)_4$CO-Asn-Phe-Phe-(D-Trp)-Lys(Boc)-Thr(But)-Phe-OTmse

Eine Lösung von 188 mg 5-Benzyloxycarbonylaminopentansäure, 847 mg rohen Heptapeptid aus der vorangehenden Stufe 4.1 und 101 mg Hydroxybenzotriazol in 4 ml Dimethylformamid wird bei 0—5° mit einer Lösung von 182 mg DCCI in 1 ml Dimethylformamid versetzt. Nach 15 Minuten bei 0—5° wird das Gemisch noch 18 Stunden bei Raumtemperatur gehalten und dann vom ausgefallenen Dicyclohexylharnstoff abfiltriert. Das Filtrat wird im Hochvakuum auf 3 ml konzentriert und das Rohprodukt durch Zugabe von 60 ml Wasser ausgefällt. Nach dem Filtrieren und Trocknen wird die Fällung zur Reinigung zweimal mit je 20 ml Methanol bei 40° verrührt und abfiltriert.

DC: [Chloroform-Methanol (85:15)] $R_f$ 0,61
[Chloroform-Methanol-Wasser (14:6:1)] $R_f$ 0,89

*Stufe 4.3*

Z—NH—$(CH_2)_4$CO-Asn-Phe-Phe-(D-Trp)-Lys(Boc)-Thr(But)-Phe-OH

760 mg von Z—NH$(CH_2)_4$CO-Asn-Phe-Phe-(D-Trp)-Lys(Boc)-Thr(But)-Phe-OTmse (Stufe 4.2) wird im 17,1 ml einer frisch hergestellten, wasserfreien 0,15M-Tetraäthylammoniumfluorid-Lösung in Dimethylformamid gelöst und 40 Minuten bei Raumtemperatur gehalten. Nach dem Abkühlen auf 0—5° wird das Reaktionsgemisch unter gutem Umrühren mit 0,51 ml 1N-wässriger Salzsäure versetzt und das Produkt durch Zugabe von 80 ml Wasser gefällt.

DC: [Chloroform-Methanol (85:15)] $R_f$ 0,13
[Chloroform-Methanol-Wasser (14:6:1)] $R_f$ 0,65

*Stufe 4.4*

$NH_2$—$(CH_2)_4$CO-Asn-Phe-Phe-(D-Trp)-Lys(Boc)-Thr(But)-Phe-OH

696 mg von Z—NH$(CH_2)_4$CO-Asn-Phe-Phe-(D-Trp)-Lys(Boc)-Thr(But)-Phe-OH (Stufe 4.3) werden in 70 ml Dimethylformamid unter Zusatz von 80 mg Pd-Kohle (10%) bis zur vollständigen Abspaltung der Z-Gruppe hydriert (ca. 2 Stunden, DC-Kontrolle). Nach dem Abfiltrieren des Katalysators wird das Filtrat im Hochvakuum auf ca. 3 ml konzentriert und das Produkt mit 50 ml peroxidfreiem Aether gefällt.

DC: [Chloroform-Methanol-Wasser (14:6:1)] $R_f$ 0,46

*Stufe 4.5*

└Asn-Phe-Phe-(D-Trp)-Lys(Boc)-Thr(But)-Phe-NH—$(CH_2)_4$CO┘

Durch Cyclisierung des linearen Octapeptids der Formel $NH_2(CH_2)_4$CO-Asn-Phe-Phe-(D-Trp)-Lys(Boc)-Thr(But)-Phe-OH (Stufe 4.4) analog dem in Beispiel 1.10 beschriebenen Verfahren erhält man mittels DCCI und N-Hydroxybenzotriazol das gewünschte cyclische Peptid. Das Rohprodukt wird durch Gegenstromverteilung (K=0,66) gereinigt.

DC: [Chloroform-Methanol (85:15)] $R_f$ 0,50
[Chloroform-Methanol-Wasser (14:6:1)] $R_f$ 0,77

## Beispiel 5

└Asn-Phe-Phe-(D-Trp)-Lys-Thr-Phe-(β-Ala)┘

in analoger Weise, wie im Beispiel 1 beschrieben, wird das geschützte Peptid der Formel

└Asn-Phe-Phe-(D-Trp)-Lys(Boc)-Thr(But)-Phe-(β-Ala)┘

mit Trifluoressigsäure behandelt und aufgearbeitet. Durch Gegenstromverteilung (K=5,0) über 170 Stufen wird das gewünschte Produkt als amorphe Substanz erhalten.

DC: System 101 $R_f$ 0,53
111B $R_f$ 0,32
112A $R_f$ 0,39

Das als Ausgangsstoff verwendete geschützte Cyclopeptid wird folgendermassen erhalten:

*Stufe 5.1*

H-(β-Ala)-OBzl . p-Toluolsulfonat

In analoger Weise wie im Beispiel 1.7A wird durch Veresterung von β-Alanin mit Benzylalkohol in Gegenwart von p-Toluolsulfonsäure das gewünschte Ester als p-Toluolsulfonat, Smp. 94,5—101°, erhalten.

DC: [Chloroform-Methanol-Wasser (14:6:1)] $R_f$ 0,38

*Stufe 5.2*

Z-Asn-Phe-Phe-(D-Trp)-Lys(Boc)-Thr(But)-Phe-(β-Ala)-OBzl

In analoger Weise wie in Beispiel 1.8 werden 1,09 g Z-Asn-Phe-Phe-(D-Trp)-Lys(Boc)-Thr(But)-Phe-OH (Beispiel 1.7) mit 0,33 g H-(β-Ala)-OBzl . p-Toluolsulfonat mittels DCCI in Gegenwart von N-Methylmorpholin und N-Hydroxybenzotriazol umgesetzt und aufgearbeitet.

DC: [Chloroform-Methanol (85:15)] $R_f$ 0,69
[Chloroform-Methanol-Wasser (14:6:1)] $R_f$ 0,89

*Stufe 5.3*

H-Asn-Phe-Phe-(D-Trp)-Lys(Boc)-Thr(But)-Phe-(β-Ala)-OH

In analoger Weise wie im Beispiel 1.9 wird der Benzylester H-Asn-Phe-Phe-(D-Trp)-Lys(Boc)-Thr(But)-Phe-(β-Ala)-OBzl (Stufe 5.2) durch Hydrierung in die entsprechende freie Säure umgewandelt.

DC: [Chloroform-Methanol-Wasser (14:6:1)] $R_f$ 0,52

*Stufe 5.4*

└Asn-Phe-Phe-(D-Trp)-Lys(Boc)-Thr(But)-Phe-(β-Ala)┘

Analog dem im Beispiel 1.10 beschriebenen Verfahren wird die freie Säure H-Asn-Phe-Phe-(D-Trp)-Lys(Boc)-Thr(But)-Phe-(β-Ala)-OH mittels DCCI und N-Hydroxybenzotriazol cyclisiert. Das Rohprodukt wird durch Gegenstromverteilung (K=0,71) gereinigt.

DC: [Chloroform-Methanol (85:15)] $R_f$ 0,65
[Chloroform-Methanol-Wasser (14:6:1)] $R_f$ 0,80

Beispiel 6

└Asn-Phe-Phe-(D-Trp)-Lys-Thr-Phe-(D-Glu)-OH

In analoger Weise, wie in Beispiel 1 beschrieben, behandelt man das geschützte Peptid der Formel └Asn-Phe-Phe-(D-Trp)-Lys(Boc)-Thr(But)-Phe-(D-Clu)-OBut mit Trifluoressigsäure und arbeitet auf. Durch Gegenstromverteilung (K=4,26) über 300 Stufen wird das gewünschte Produkt als amorphe Substanz erhalten.

DC: 101 $R_f$ 0,53
IIB $R_f$ 0,40
112A $R_f$ 0,31

(Das Symbol —Glu-OH representiert den Rest der Formel

—NH—CH—$(CH_2)_2$—CO—.)
|
CO—OH

Das als Ausgangsstoff verwendete geschützte Peptid wird folgendermassen erhalten:

*Stufe 6.1*

H-[D-Glu(OBzl)]-OBut . Hydrochlorid

Ein Gemisch von 5,00 g H-[D-Glu(OBzl)]-OH und 40 ml flüssigem Isobuten wird mit 4 ml konzentrierter Schwefelsäure in 40 ml Dioxan in einem verschlossenen Gefäss bei Raumtemperatur gerührt, bis eine klare Lösung entsteht, und weitere 4 Stunden bei Raumtemperatur belassen. Zur Aufarbeitung wird das auf −20° abgekühlte Reaktionsgemisch in eine eiskalte Mischung von 300 ml Aether und 203 ml 1-N Natriumhydroxid gegossen. Die Aetherphase wird dreimal mit je 50 ml Wasser gewaschen.

Nach dem Nachextrahieren der wässerigen Phase mit weiteren 300 ml Aether werden die vereinigten organischen Phasen über Natriumsulfat getrocknet, im Vakuum auf 10 ml eingeengt und bei 0° unter gutem Umrühren mit 30 ml 0,7-N Chlorwasserstoff-Lösung in Methanol versetzt. Die erhaltene Lösung wird im Vakuum eingedampft und der Rückstand mit 20 ml Petroläther verrieben, bis Kristallisation eintritt. Das abfiltrierte Produkt der Formel

CO-OBut
|
$HCl . NH_2—CH(CH_2)_2—CO-OBzl$

wird durch Chromatographie an einer Kieselgelsäule (170 g) mittels Chloroform-Methanol (85:15) gereinigt, Smp. 107—108° (Zersetzung), $[\alpha]_D^{25}$: $-15° \pm 1°$ (Aethanol, 2%).
DC [Chloroform-Methanol (85:15)] $R_f$ 0,60

*Stufe 6.2*
Z-Asn-Phe-Phe-(D-Trp)-Lys(Boc)-Thr(But)-Phe-D-Glu(OBzl)-OBut

Eine Lösung von 1,09 g Z-Asn-Phe-Phe-(D-Trp)-Lys(Boc)-Thr(But)-Phe-OH (Beispiel 1.7), 0,31 g HCl · [D-Glu(OBzl)]-OBut (Stufe 6.1), 0,13 g N-Hydroxybenzotriazol und 95 mg N-Methylmorpholin in 8 ml Dimethylformamid wird bei 0—5° mit einer Lösung von 0,21 g DCCl in 2 ml Dimethylformamid versetzt und nach 30 Minuten bei 0—5° noch 15 Stunden bei Zimmertemperatur gehalten. Der ausgefallene Dicyclohexylharnstoff wird abfiltriert und das Filtrat im Hochvakuum eingedämpft. Der Rückstand wird mit 30 ml Wasser verrieben, filtriert, getrocknet und zur Reinigung noch zweimal mit je 10 ml Methanol verrührt und abfiltriert.
DC: [Chloroform-Methanol (85:15)] $R_f$ 0,85
[Chloroform-Methanol-Wasser (14:6:1)] $R_f$ 0,91

*Stufe 6.3*
H-Asn-Phe-Phe-(D-Trp)-Lys(Boc)-Thr(But)-Phe-[D-Glu]-OBut

In analoger Weise wie im Beispiel 1.9 wird die Verbindung der Formal H-Asn-Phe-Phe-(D-Trp)-Lys(Boc)-Thr(But)-Phe-[D-Glu(OBzl)]-OBut (Stufe 6.2) zum gewünschten Produkt hydriert.
DC: [Chloroform-Methanol (85:15)] $R_f$ 0,13
[Chloroform-Methanol-Wasser (14:6:1)] $R_f$ 0,65

*Stufe 6.4*
Asn-Phe-Phe-(D-Trp)-Lys(Boc)-Thr(But)-Phe-[D-Glu]-OBut (cyclisch verbunden)

In analoger Weise, wie im Beispiel 1.10 beschrieben, wird die Säure der Formel H-Asn-Phe-Phe-(D-Trp)-Lys(Boc)-Thr(But)-Phe-[D-Glu]-OBut mittels DCCl und N-Hydroxybenzotriazol cyclisiert. Das Rohprodukt wird durch Gegenstromverteilung (K=0,52) gereinigt.
DC: [Chloroform-Methanol (85:15)] $R_f$ 0,54
[Chloroform-Methanol-Wasser (14:6:1)] $R_f$ 0,83

Beispiel 7

Phe-Phe-(D-Trp)-Lys-Thr-Phe (cyclisch verbunden)

235 mg geschütztes Hexapeptid der Formel Phe-Phe-(D-Trp)-Lys(Boc)-Thr(But)-Phe (cyclisch verbunden) werden analog Beispiel 1 mit Trifluoressigsäure von den Schutzgruppen befreit und als Acetat durch Gegenstromverteilung im gleichen System wie im Beispiel 1 gereinigt. Die reine Substanz wird bie K=5,4 isoliert.

DC (Kieselgel, Merck): System 45: $R_f$ 0,2
52: $R_f$ 0,35
157: $R_f$ 0,5

Das Ausgangsmaterial wird wie folgt erhalten:

*Stufe 7.1*
Z-Phe-Phe-(D-Trp)-Lys(Boc)-Thr(But)-Phe-OTmse

Eine Lösung von 2,84 g H-(D-Trp)-Lys(Boc)-Thr(But)-Phe-OTmse (Beispiel 1.5) und 1,52 g Z-Phe-Phe-OH in 12 ml Dimethylformamid werden bei 0° mit 0,62 g N-Hydroxybenzotriazol und 0,98 g DCCl versetzt und 16 Stunden bei 0° belassen. Nach Abfiltrieren des Dicyclohexylharnstoffs wird das Produkt durch Eintropfen in verdünnte Natriumhydrogencarbonatlösung gefällt. Das Produkt wird aus wässrigem Methanol umkristallisiert.
DC (Kieselgel, Merck): System 157A, $R_f$ 0,6.

*Stufe 7.2*

Z-Phe-Phe-(D-Trp)-Lys(Boc)-Thr(But)-Phe-OH

Eine Lösung von 2,86 g Z-Phe-Phe-(D-Trp)-Lys(Boc)-Thr(But)-Phe-OTmse in 13,5 ml Dimethylformamid wird bei 30° mit 6,1 ml einer 1,12-M Lösung von Tetrabutylammoniumfluorid in Dimethylsulfoxid versetzt und 5 Minuten bei dieser Temperatur belassen. Das Produkt wird durch Eintropfen in eiskalte verdünnte Salzsäure gefällt und durch Umfällen aus Acetonitril-Lösung mit verdünnter Salzsäure gereinigt.

DC: System 157A $R_f$ 0,35

*Stufe 7.3*

└Phe-Phe-(D-Trp)-Lys(Boc)-Thr(But)-Phe┘

Eine Lösung von 870 mg Z-Phe-Phe-(D-Trp)-Lys(Boc)-Thr(But)-Phe-OH in 20 ml Dimethylformamid wird in Gegenwart von 90 mg Palladium-Kohle (10%) 2 Stunden bei Raumtemperatur hydriert. Nach Abfiltrieren des Katalysators wird das Filtrat im Hochvakuum auf wenige ml konzentriert, mit 790 ml Dimethylformamid, 1,2 g N-Hydroxybenzotriazol und 1,6 g DCCI versetzt und 15 Stunden bei 50° belassen. Nach Zugabe von 2 ml 5-M Oxalsäurelösung in Dimethylformamid wird im Hochvakuum auf ca. 10 ml eingeengt, der Dicyclohexylharnstoff abgenutscht und das Produkt durch Eintropfen in verdünnte Natriumhydrogencarbonatlösung gefällt. Durch Chromatographie an Kieselgel wird das reine Produkt mit Chloroform unter Zusatz von 3—5% Methanol eluiert.

DC: System 157A $R_f$ 0,5

Beispiel 8

└Phe-Phe-(D-Trp)-Lys-Thr-Phe-Gly┘

450 mg └Phe-Phe-(D-Trp)-Lys(Boc)-Thr(But)-Phe-Gly┘ werden, wie im Beispiel 1 beschrieben, mit Trifluoressigsäure behandelt. Das rohe Produkt wird mit einem Ionenaustauscher in das Acetat übergeführt und dieses durch Gegenstromverteilung im System n-Butanol-Essigsäure-Wasser-Toluol (4:1:5:4) über 420 Stufen gereinigt. Die reine Substanz (K=1,5) wird in üblicher Weise isoliert.

DC (Kieselgel, Merck): System 157C $R_f$ 0,25

Das Ausgangsmaterial wird folgendermassen erhalten:

*Stufe 8.1*

Z-Phe-Phe-(D-Trp)-Lys(Boc)-Thr(But)-Phe-Gly-OBzl

Eine Lösung von 800 mg Z-Phe-Phe-(D-Trp)-Lys(Boc)-Thr(But)-Phe-OH (Beispiel 7.2), 290 mg H-Gly-OBzl . p-Toluolsulfonat und 130 mg N-Hydroxybenzotriazol in 4 ml Dimethylformamid wird bei 0° mit 0,095 ml N-Methylmorpholin und 200 mg DCCI versetzt und 16 Stunden bei 0° belassen. Nach Abfiltrieren des Dicyclohexylharnstoffs wird das Produkt durch Eintropfen in verdünnte Natriumhydrogencarbonatlösung gefällt. Das Rohprodukt wird durch Umkristallisieren aus Methanol-Wasser (9:1) und Acetonitrile-Wasser (1:1) gereinigt.

DC: [Chloroform-Methanol (95:5)]:$R_f$ 0,55

System 157A: $R_f$ 0,75

*Stufe 8.2*

└Phe-Phe-(D-Trp)-Lys(Boc)-Thr(But)-Phe-Gly┘

Eine Lösung von 780 mg Z-Phe-Phe-(D-Trp)-Lys(Boc)-Thr(But)-Phe-Gly-OBzl (Stufe 8.1) in 15 ml Dimethylformamid wird in Gegenwart von 80 mg Palladium-Kohle (10%) während 6 Stunden hydriert. Nach Abfiltrieren des Katalysators wird das Filtrat stark eingeengt, mit 115 ml Dimethylformamid, 910 mg N-Hydroxybenzotriazol und 1,22 g DCCI versetzt und 20 Stunden bei 50° belassen. Die Aufarbeitung erfolgt, wie im Beispiel 7.3 beschrieben. Zur Reinigung des Rohprodukts wird eine Gegenstromverteilung über 460 Stufen im gleichen System, wie im Beispiel 1.10 beschrieben, durchgeführt. (K=0,4)

DC: System 157A: $R_f$ 0,4

Beispiel 9

└Phe-Phe-(D-Trp)-Lys-Thr-Phe-Gaba┘

260 mg └Phe-Phe-(D-Trp)-Lys(Boc)-Thr(But)-Phe-Gaba┘ werden analog Beispiel 1 mit Trifluoressigsäure behandelt. Mittels Ionenaustauscher wird das Produkt in das Acetat übergeführt, welches durch Gegenstromverteilung über 300 Stufen im System sek-Butanol-Wasser-Essigsäure (100:100:1)

gereinigt wird. Die reine Substanz (K = 1,5) wird wie üblich isoliert.
DC: (Kieselgel, Merck): System 157 $R_f$ 0,5
Das Ausgangsmaterial wird folgendermassen erhalten:

*Stufe 9.1*
Z-Phe-Phe-(D-Trp)-Lys(Boc)-Thr(But)-Phe-Gaba-OBzl

Eine Lösung von 420 mg Z-Phe-Phe-(D-Trp)-Lys(Boc)-Thr(But)-Phe-OH (Beispiel 7.2), 165 mg H-Gaba-OBzl.p-Toluolsulfonat und 70 mg N-Hydroxybenzotriazol in 2 ml Dimethylformamid wird bei 0° mit 0,05 ml N-Methylmorpholin und 100 mg DCCI versetzt und 20 Stunden bei 0° belassen. Nach Abfiltrieren des Dicyclohexylharnstoffs wird das Produkt durch Eintropfen in Natriumhydrogencarbonatlösung ausgefällt.
DC: System 157A: $R_f$ 0,65

*Stufe 9.2*
Phe-Phe-(D-Trp)-Lys(Boc)-Thr(But)-Phe-Gaba

Eine Lösung von 500 mg Z-Phe-Phe-(D-Trp)-Lys(Boc)-Thr(But)-Phe-Gaba-OBzl in 20 ml Dimethylformamid wird in Gegenwart von 50 mg Palladium-Kohle (10%) 2 Stunden hydriert. Nach Abfiltrieren des Katalysators wird das Filtrat im Hochvakuum stark eingeengt, der Rückstand mit 370 ml Dimethylformamid, 560 mg N-Hydroxybenzotriazol und 760 mg DCCI versetzt und 18 Stunden bei 50° belassen. Die Aufarbeitung und Reinigung des Produkts erfolgt analog Beispiel 8.2, K = 0,3
DC: System 157A: $R_f$ 0,45

## Beispiel 10

Asn-Phe-Phe-(D-Trp)-Lys-Thr-Phe-Gly

600 mg Asn-Phe-Phe-(D-Trp)-Lys(Boc)-Thr(But)-Phe-Gly werden, wie im Beispiel 1 beschrieben, mit Trifluoroessigsäure behandelt, aufarbeitet und gereinigt, K = 3,5.
DC: System 157C: $R_f$ 0,15
Das Ausgangsmaterial wird wie folgt erhalten:

*Stufe 10.1*
Z-Asn-Phe-Phe-(D-Trp)-Lys(Boc)-Thr(But)-Phe-Gly-OBzl

Ein Lösung von 230 mg Z-Asn-Phe-(D-Trp)-Lys(Boc)-Thr(But)-Phe-OH (Beispiel 1.7), 77 mg H-Gly-OBzl.p-Toluolsulfonat und 35 mg N-Hydroxybenzotriazol in 1 ml Dimethylformamid wird bei 0° mit 0,025 ml N-Methylmorpholin und 53 mg DCCI versetzt. Nach 16 Stunden wird analog Beispiel 7.1 aufgearbeitet und aus Acetonitril-Wasser (4:1) umkristallisiert.
DC: System 157A: $R_f$ 0,65

*Stufe 10.2*
Asn-Phe-Phe-(D-Trp)-Lys(Boc)-Thr(But)-Phe-Gly

Eine Lösung von 200 mg Z-Asn-Phe-Phe-(D-Trp)-Lys(Boc)-Thr(But)-Phe-Gly-OBzl (Stufe 10.1) in 15 ml Dimethylformamid wird in Gegenwart von 50 mg Palladium-Kohle (10%) hydriert. Nach Abfiltrieren des Katalysators wird das Filtrat im Hochvakuum eingeengt, der Rückstand mit 140 ml Dimethylformamid, 215 mg N-Hydroxybenzotriazol und 290 mg DCCI versetzt und 20 Stunden bei 50° belassen. Die Aufarbeitung und Reinigung erfolgt wie im Beispiel 8.2, K = 0,8.
DC: System 157B: $R_f$ 0,45

## Beispiel 11

Asn-Phe-Phe-[D-Trp(F)]-Lys-Thr-Phe-Gaba

340 mg Asn-Phe-Phe-[D-Trp(F)]-Lys(Boc)-Thr(But)-Phe-Gaba werden, wie im Beispiel 1 beschrieben, mit Trifluoressigsäure behandelt, mittels Ionenaustauscher in das Acetat übergeführt und durch Gegenstromverteilung im System tert-Butanol-Toluol-Methanol-Puffer (0,05M Ammoniumacetat + 0,05M Essigsäure) (7:7:3:10) über 500 Stufen (K = 0,14) gereinigt.
(Das Symbol [D-Trp(F)] bedeutet 5-Fluor-D-tryptophan.)
DC: System 157: $R_f$ 0,36
Das Ausgangsmaterial wird folgendermassen erhalten:

*Stufe 11.1*
Z-[D-Trp(F)]-OH

Eine Lösung von 2 g 5-Fluor-DL-tryptophan in 9 ml 1-N Natronlauge wird unter Eiskühlung und

starkem Rühren gleichzeitig mit 1,4 ml Chlorameisensäurebenzylester und 10,5 ml 1N Natronlauge versetzt. Nach weiteren 2 Stunden werden 50 ml Aethylacetat zugefügt, pH des zweiphasigen Gemisches mit Salzsäure auf 1 gestellt, die organische Phase abgetrennt, mit Wasser gewaschen, über Natriumsulfat getrocknet und abgedampft. Der Rückstand wird in 9,5 ml Wasser und 5 ml 2-N Natronlauge gelöst, nach Zugabe von 1,5 ml Anilin, 100 mg L-Cystein-hydrochlorid und 34 ml 0,2M Citratpuffer(pH 5)fällt des Ausgangsmaterial zum Teil wieder aus; die Suspension wird mit 2-N Salzsäure auf pH 6,3 gebracht. 260 mg Papain werden in 2 ml Wasser von 40° aufgeschlämmt, das Unlösliche abzentrifugiert, 1,9 ml der überstehenden Lösung zu der obigen Suspension gegeben und diese 20 Stunden bei 40° gerührt. Unter Eiskühlung wird das pH des Gemisches mit 2-N Natronlauge auf 8 gebracht, das unlösliche Z-[L-Trp(F)]-anilid abfiltriert, das Filtrat mit 5N Salzsäure auf pH 5,3 gestellt und wie oben mit Cystein-hydrochlorid und Papain versetzt. Nach weiteren 20 Stunden wird das gebildete Anilid wieder abfiltriert, und das Filtrat nochmals während 3 Tagen gleich wie oben behandelt, wodurch nur noch Spuren des Anilids gebildet werden. Das Filtrat wird mit Aethylacetat überschichtet, auf pH 1-2 angesäuert; die organische Schicht wird mit Wasser gewaschen, über Natriumsulfat getrocknet und eingedämpft. Der Rückstand wird aus Diisopropyläther-Hexan umkristallisiert.
DC: System 157B: $R_f$ 0,3

*Stufe 11.2*
Z-[D-Trp(F)]-Lys(Boc)-OH

1,15 g Z-[D-Trp(F)]-OH wird gemäss dem in Beispiel 1.3A beschriebenen Verfahren in das im Titel genannten Dipeptid übergeführt.
DC: System 157B: $R_f$ 0,33

*Stufe 11.3*
Z-[D-Trp(F)]-Lys(Boc)-Thr(But)-Phe-OTmse

Eine Lösung von 380 mg Z-[D-Trp(F)]-Lys(Boc)-OH, 300 mg H-Thr(But)-Phe-OTmse (Beispiel 1.3) und 110 mg N-Hydroxy-benzotriazol in 2,5 ml Dimethylformamid wird bei 0° mit 170 mg DCCI versetzt. Nach 15 Stunden wird analog Beispiel 7.1 aufgearbeitet und das Produkt durch Umkristallisieren aus Aethylacetat-Petroläther gereinigt.
DC: System 157A: $R_f$ 0,76

*Stufe 11.4*
Z-Asn-Phe-Phe-[D-Trp(F)]-Lys(Boc)-Thr(But)-Phe-OTmse

Eine Lösung von 490 mg Z-[D-Trp(F)]-Lys(Boc)-Thr(But)-Phe-OTmse in 15 ml Methanol-Wasser (9:1) wird in Gegenwart von 50 mg Palladium-Kohle (10%) hydriert, wobei durch eine gleichzeitige Zugabe von 0,2N Salzsäure im obgenannten Lösungsmittel pH 5 durchlaufend gehalten wird. Das Reaktionsgemisch wird filtriert, das Filtrat eingeengt und dreimal durch Abdampfen mit Dimethylformamid entwässert. Zu der verbliebenen Lösung (1,2 g) werden 305 mg Z-Asn-Phe-Phe-OH, 83 mg N-Hydroxybenzotriazol, 0,06 ml N-Methylmorpholin und 1,2 ml Dimethylformamid gegeben. Bei −5° gibt man 130 mg DCCI zu und belässt 16 Stunden bei dieser Temperatur. Nach der Aufarbeitung analog Beispiel 7.1 wird das Produkt aus Acetonitril-Wasser umkristallisiert.
DC: System 157A: $R_f$ 0,6

*Stufe 11.5*
Z-Asn-Phe-Phe-[D-Trp(F)]-Lys(Boc)-Thr(But)-Phe-OH

Eine Lösung von 600 mg Z-Asn-Phe-Phe-[D-Trp(F)]-Lys(Boc)-Thr(But)-Phe-OTmse in 4,2 ml Dimethylformamid wird bei 30° mit 3,8 ml 0,34-M Tetraäthylammoniumfluorid-Lösung in Dimethylsulfoxid versetzt und 30 Minuten bei 30° belassen. Die Lösung wird unter Eiskühlung in 50 ml Wasser und 0,65 ml 2-N Salzsäure getropft und die Fällung abfiltriert. Das Produkt wird durch Umkristallisieren aus Acetonitril-Wasser gereinigt.
DC: System 157A: $R_f$ 0,35

*Stufe 11.6*
Z-Asn-Phe-Phe-[D-Trp(F)]Lys(Boc)-Thr(But)-Phe-Gaba-OBzl

Eine Lösung von 470 mg Z-Asn-Phe-Phe-[D-Trp(F)]-Lys(Boc)-Thr(But)-Phe-OH, 165 mg H-Gaba-OBzl.p-Toluolsulfonat (Beispiel 1.7A) und 66 mg N-Hydroxybenzotriazol in 2 ml Dimethylformamid wird bei −5° mit 0,05 ml N-Methylmorpholin und 100 mg DCCI versetzt. Nach 16 Stunden bei dieser Temperatur wird analog Beispiel 7.1 aufgearbeitet. Das Produkt wird, bis auf Spuren von Dicyclohexylharnstoff, rein erhalten.
DC: System 157A: $R_f$ 0,55

*Stufe 11.7*

Asn-Phe-Phe-[D-Trp(F)]-Lys(Boc)-Thr(But)-Phe-Gaba

Eine Lösung von 520 mg Z-Asn-Phe-Phe-[D-Trp(F)]-Lys(Boc)-Thr(But)-Phe-Gaba-OBzl in 20 ml

Dimethylformamid wird in Gegenwart von 50 mg Palladium-Kohle (10%) 2 Stunden hydriert. Nach Abfiltrieren des Katalysators wird das Filtrat im Hochvakuum eingeengt und mit 350 ml Dimethylformamid, 540 mg N-Hydroxybenzotriazol und 720 mg DCCI versetzt. Nach 20 Stunden bei 50° wird analog Beispiel 8.2 aufgearbeitet und gereinigt; K = 1,1.
DC: System 157A: $R_f$ 0,35

Beispiel 12

└─ Asn-Phe-Phe-[D-Trp($NO_2$)]-Lys-Thr-Phe-Gaba ─┘

In analoger Weise, wie im Beispiel 1 beschrieben, behandelt man das geschützte Peptid der Formel └─Asp-Phe-Phe-[D-Trp($NO_2$)]-Lys(Boc)-Thr(But)-Phe-Gaba─┘ mit Trifluoressigsäure und arbeitet auf. Durch Gegenstromverteilung über 220 Stufen (K = 4,5) wird das gewünschte Produkt als eine dünnschichtchromatographisch einheitliche amorphe Substanz erhalten.
DC: System 101 : $R_f$ 0,58
111B: $R_f$ 0,40
112A: $R_f$ 0,45

(Das Symbol [D-Trp($NO_2$)] bedeutet 6-Nitro-D-tryptophan.) Das Ausgangsmaterial wird folgendermassen erhalten:

*Stufe 12.1*
Z-Gaba-OTmse

Eine Lösung von 5,93 g Z-Gaba-OH und 3,84 g Trimethyl-silyl-äthanol in 10 ml Acetonitril und 6 ml Pyridin wird auf 5° abgekühlt und unter Rühren mit 5,70 g DCCI versetzt. Nach 1 Stunde bei 5° wird das Reaktionsgemisch noch während 15 Stunden bei Raumtemperatur gehalten. Der ausgefallene Dicyclohexylharnstoff wird abfiltriert und mit Aethylacetat gewaschen, und das Filtrat eingedampft. Der Rückstand wird auf eine Kieselgelsäule (350 g) chromatographiert, wobei ein Gemisch von Tetrachlorkohlenstoff-Aethylacetat (6:4) als Eluiermittel dient. Das Produkt enthaltende Fraktionen werden zusammengefasst und eingedampft. Das erhaltene farblose Oel wird im Hochvakuum getrocknet.
DC: [Chloroform-Methanol (85:15)] $R_f$ 0,69
[Toluol-Aceton (7:3)] $R_f$ 0,59

*Stufe 12.2*
H-Gaba-OTmse

Durch eine Lösung von 4,70 g Z-Gaba-OTmse (Stufe 12.1) in 50 ml Isopropanol wird nach Zugabe von 0,5 g Palladium-Kohle (10%) Wasserstoff geleitet, bis dünnschichtchromatographisch [Chloroform-Methanol (85:15)] kein Ausgangsstoff mehr nachweisbar ist. Der Katalysator wird abfiltriert und das Filtrat im Vakuum eingedampft. Das erhaltene oelige Rohprodukt wird direkt für die nächste Stufe eingesetzt.

*Stufe 12.3*
Z-Phe-Gaba-OTmse

Zu einer Lösung von 4,60 g Z-Phe-OH und 2,83 g H-Gaba-OTmse (Stufe 12.2) in 15 ml Methylenchlorid wird bei 0—5° eine Lösung von 3.75 g Dicyclohexylcarbodiimid in 5 ml Methylenchlorid zugegeben. Das Reaktionsgemisch wird noch 15 Minuten bei 5° und anschliessend 2 Stunden bei Raumtemperatur gehalten, vom ausgefallenen DCH abfiltriert, und das Filtrat eingedampft. Der Rückstand wird zur Reinigung dreimal aus Aethylacetat (12 ml) und Petroläther (60 ml) umkristallisiert, Smp. 88—93,5° (Zersetzung). (DCH = Dicyclohexylharnstoff)
DC: [Chloroform-Methanol (85:15)] $R_f$ 0,72
[Toluol-Aceton (7:3)] $R_f$ 0,44

*Stufe 12.4*
Z-Thr(But)-Phe-Gaba-OTmse

Eine Lösung von 3,93 g H-Phe-Gaba-OTmse [erhalten aus 5,42 g Z-Phe-Gaba-OTmse (Stufe 12.3) durch Hydrierung analog Stufe 12.2] und 5,45 g Z-Thr(But)-ONSu in 25 ml Dimethylformamid wird während 15 Stunden bei Raumtemperatur gehalten, mit 0,34 ml Dimethylaminopropylamin versetzt und eine weitere Stunde bei Raumtemperatur belassen. Das Reaktionsgemisch wird im Hochvakuum eingedampft, der Rückstand in etwa 200 ml Aethylacetat gelöst und dreimal mit je 30 ml einer 5%-igen wässrigen Weinsäure-Lösung und dreimal mit je 30 ml Wasser gewaschen. Die über Natriumsulfat getrocknete organische Phase wird im Vakuum eingedampft und der Rückstand zweimal aus Aethylacetat (10 ml)-Petroläther (80 ml) umkristallisiert, Smp. 114—117° (Zersetzung).
DC: [Chloroform-Methanol (85:15)] $R_f$ 0,75
[Tetrachlorkohlenstoff-Aethylacetat (6:4)] $R_f$ 0,39

*Stufe 12.5*

Z-Lys(Boc)-Thr(But)-Phe-Gaba-OTmse

Zu einer Lösung von 2,37 g H-Thr(But)-Phe-Gaba-OTmse [erhalten aus 3,00 g Z-Thr(But)-Phe-Gaba-OTmse durch Hydrierung analog Stufe 12.2], 1,96 g Z-Lys(Boc)-OH und 0,70 g N-Hydroxybenzotriazol in 10 ml Methylenchlorid wird bei 0—5° eine Lösung von 1,25 g DCCI in 5 ml Methylenchlorid zugefügt. Nach 15 Minuten bei 0—5° wird das Gemisch während 15 Stunden bei Raumtemperatur gehalten. Der ausgefallene Dicyclohexylharnstoff wird abfiltriert, das Filtrat im Vakuum eingedampft, und der Rückstand dreimal aus Aethylacetat (20 ml)-Petroläther (100 ml) umgefällt.

DC: [Chloroform-Methanol (85:15)] $R_f$ 0,73
[Chloroform-Methanol-Wasser (14:6:1)] $R_f$ 0,87

*Stufe 12.5A*

Bpoc-Phe-[D-Trp($NO_2$)]-OH

Eine Suspension von 0,50 g 6-Nitro-D-tryptophan und 1,10 g Bpoc-Phe-ONSu in 10 ml eines Gemisches von Dimethylformamid-Wasser (8:2) wird bei Raumtemperatur mit 0,1-N Natronlauge so behandelt, dass pH auf einem Wert von 7,5 bleibt. Nach 2,5 Stunden beträgt der Gesamtverbrauch der Natronlauge 31,5 ml. Das Reaktionsgemisch wird auf 0—5° abgekühlt und langsam mit 3,5 ml 1-N Salzsäure versetzt. Das ausgefallene Rohprodukt wird abfiltriert, getrocknet und zweimal durch eine Kieselgelsäule (jeweils 60 g Adsorbens) mit einem Gemisch von Chloroform-Methanol (85:15) und Tetrachlorkohlenstoff-Aethylacetat (6:4) als Eluiermittel chromatographiert.

DC: [Chloroform-Methanol (85:15)] $R_f$ 0,12
[Chloroform-Methanol-Wasser (14:6:1)] $R_f$ 0,50

*Stufe 12.6*

Bpoc-Phe-[D-Trp($NO_2$)]-Lys(Boc)-Thr(But)-Phe-Gaba-OTmse

Zu einer Lösung von 1,08 g H-Lys(Boc)-Thr(But)-Phe-Gaba-OTmse [erhalten aus 1,30 g Z-Lys(Boc)-Thr(But)-Phe-Gaba-OTmse (Stufe 12.5) durch Hydrierung analog Stufe 12.2] 0,93 g Bpoc-Phe-[D-Trp($NO_2$)]-OH (Stufe 12.5A) und 0,22 g N-Hydroxybenzotriazol in 8 ml Methylenchlorid wird bei 0—5° eine Lösung von 0,36 g DCCI in 2 ml Methylenchlorid zugefügt. Nach 15 Minuten bei 0—5° wird das Gemisch während 4 Stunden bei Raumtemperatur gehalten. Zur Aufarbeitung wird der ausgefallene Dicyclohexylharnstoff abfiltriert, das Filtrat im Vakuum eingedampft und der Rückstand aus einem Gemisch von Aethylacetat (10 ml) und Petroläther (70 ml) umgefällt und anschliessend mit Methanol (10 ml) verrieben.

DC: [Toluol-Aceton (7:3)] $R_f$ 0,20
[Chloroform-Methanol-Wasser (14:6:1)] $R_f$ 0,90

*Stufe 12.7*

HCl.H-Phe-[D-Trp($NO_2$)]-Lys(Boc)-Thr(But)-Phe-Gaba-OTmse

Eine Lösung von 1,27 g Bpoc-Asn-Phe-Phe[D-Trp($NO_2$)]-Lys(Boc)-Thr(But)-Phe-Gaba-OTmse (Stufe 12.6) in einem Gemisch von 27 ml Trifluoräthanol und 3 ml Wasser wird durch allmähliche Zugabe einer Mischung von Trifluoräthanol mit konzentrierter Salzsäure (9:1) bei scheinbarem pH 1,5 gehalten (Glaselektrode, automatisches Titriergerät. Bis zum Abschluss der Reaktion wird während 1,5 Stunden bei Raumtemperatur ca. 0,76 ml des Reagens verbraucht. Das nach dem Eindampfen im Vakuum erhaltene Rohprodukt wird während einer Stunde mit 30 ml Aether verührt und dann abfiltriert.

DC: [Chloroform-Methanol (85:15)] $R_f$ 0,55

*Stufe 12.7A*

2-Trimethylsilyläthyl-N-hydroxy-succinimidocarbonat (SEOC-ONsu)

Einer Lösung von 100 g Chloramaeisensäure-N-hydroxy-succinimidester und 73,2 g 2-Trimethylsilyläthanol in 160 ml Methylenchlorid wird bei 0—5° 62,6 g N-Methylmorpholin innerhalb 15 Minuten zugetropft. Das Gemisch wird während 2 Stunden bei 0—5° verrührt, zur Aufarbeitung mit 1 Liter Aether versetzt und mit je zweimal 200 ml 1-N HCl, 200 ml Wasser, 200 ml 5%-iger Natriumhydrogencarbonat-Lösung und dreimal 200 ml Wasser gewaschen. Die wässrigen Phasen werden mit 0,5 Liter Aether nachextrahiert und die vereinigten organischen Phasen über Natriumsulfat getrocknet und eingedampft. Der Rückstand wird aus 360 ml Diisopropyläther umgelöst und die entstandenen Kristalle abfiltriert und mit 150 ml Hexan gewaschen, Smp. 101—102°.

*Stufe 12.7B*

SEOC-Asn-OH

In einer Lösung von 2,64 g L-Asparagin in 80 ml eines Gemisches von Dimethylformamid-Wasser (6:2) wird das pH auf 7.5 durch Zugabe von 0,21 ml 1-N Natronlauge gestellt, und dann, stets unter Aufrechterhaltung von pH 7,5 durch Zugabe weiterer Mengen 1-N Natronlauge mittels eines automatischen Titriergerätes, eine Lösung von 5,19 g SEOC-ONSu (Stufe 12.7A) in 10 ml Dimethylformamid

innerhalb einer Stunde zugetropft. Nach 2,5 Stunden wird eine kleine Menge ungelöstes Material abfiltriert und das Filtrat mit einer Menge verdünnter Salzsäure versetzt, die der Menge verbrauchter 1-N Natronlauge (ca. 20 ml) äquivalent ist. Die Lösung wird im Hochvakuum eingedampft und der Rückstand mit 30 ml kaltem Wasser verrieben und filtriert, Smp. 144,5—146,5° (Zersetzung).
DC: [Chloroform-Methanol-Wasser (14:6:1)] $R_f$ 0,15

*Stufe 12.7C*
SEOC-Asn-ONSu

Einer Lösung von 1,72 g SEOC-Asn-OH (Stufe 12.7B) in einem Gemisch von 20 ml Aethylacetat und 10 ml Dimethylformamid wird 0,79 g N-Hydroxy-succinimid zugeführt und die auf 0—5° abgekühlte Lösung mit 1,54 g DCCI versetzt. Nach 2 Stunden bei 0—5° wird der ausgefallene Dicyclohexylharnstoff abfiltriert, das Filtrat mit 150 ml Aethylacetat verdünnt und dreimal mit je 50 ml 1%-iger Oxalsäurelösung und viermal mit je 50 ml Wasser gewaschen. Die organische Phase wird über Natriumsulfat getrocknet und im Vakuum eingedampft; der erhaltene Schaum wird ohne weitere Reinigung für die nächste Stufe verwendet.
DC: [Chloroform-Methanol-Wasser (14:6:1)] $R_f$ 0,75

*Stufe 12.7D*
SEOC-Asn-Phe-OH

Einer Suspension von 0,64 g Phenylalanin 1,44 g SEOC-Asn-ONSu (Stufe 12.7C) in einem Gemisch von 5 ml Dimethylformamid und 5 ml Wasser wird während einer halben Stunde bei Raumtemperatur 1-N Natronlauge mittels eines automatischen Titriergerätes mit einer solchen Geschwindigkeit zugetropft, dass das pH von 7,5 eingehalten wird. Zur Aufarbeitung wird eine kleine Menge ungelöstes Material abfiltriert und das Filtrat bei 0—5° mit einer Menge verdünnter Salzsäure (ca. 4,2 ml einer 1-N Lösung) verstezt, die der Menge verbrauchter Natronlauge äquivalent ist. Das ausgefallene Rohprodukt wird über Phosphorpentoxid getrocknet und aus 40 ml Aethylacetat umkristallisiert; Smp. 156—158° (Zersetzung).
DC: [Chloroform-Methanol-Wasser (14:6:1)] $R_f$ 0,33

*Stufe 12.8*
SEOC-Asn-Phe-Phe-[D-Trp($NO_2$)]-Lys(Boc)-Thr(But)-Phe-Gaba-OTmse

Eine Lösung von 500 mg HCl.H-Phe-[D-Trp($NO_2$)]-Lys(Boc)-Thr(But)-Phe-Gaba-OTmse (Stufe 12.7), 202 mg SEOC-Asn-Phe-OH (Stufe 12.7D), 65 mg N-Hydroxybenzotriazol und 44 mg N-Methylmorpholin in 5 ml Dimethylformamid wird auf 0° abgekühlt und mit 116 mg DCCI versetzt, 15 Minuten bei 0—5° und 6 Stunden bei Raumtemperatur gehalten. Der ausgefallene Dicyclohexylharnstoff wird abfiltriert, das Filtrat im Hochvakuum eingedampft und der Rückstand mit 20 ml Wasser verrieben und abgenutscht. Das Rohprodukt wird noch zweimal mit je 4 ml Methanol verrieben und abfiltriert.
DC: [Chloroform-Methanol (85:15)] $R_f$ 0,80
[Chloroform-Methanol-Wasser (14:6:1)] $R_f$ 0,90

*Stufe 12.9*
H-Asn-Phe-Phe-[D-Trp ($NO_2$)]-Lys(Boc)-Thr(But)-Phe-Gaba-OH

530 mg SEOC-Asn-Phe-Phe-[D-Trp($NO_2$)]-Lys(Boc)-Thr(But)-Phe-Gaba-Tmse (Stufe 12.8) wird in 46,7 ml einer frisch hergestellten, wasserfreien 0,15 M-Tetraäthylammoniumfluorid-Lösung in Dimethylformamid gelöst und während einer Stunde bei Raumtemperatur gehalten. Die Lösung wird auf 0—5° abgekühlt, unter gutem Umrühren mit 0,70 ml wässriger 1-N Salzsäure versetzt und im Hochvakuum auf 3 ml konzentriert. Das Produkt wird durch Zugabe von 30 ml Wasser gefällt.
DC: [Chloroform-Methanol-Wasser (14:6:1)] $R_f$ 0,45

*Stufe 12.10*

└Asn-Phe-Phe-[D-Trp($NO_2$)]-Lys(Boc)-Thr(But)-Phe-Gaba┘

In einer analogen Weise, wie im Beispiel 1 beschrieben, wird H-Asn-Phe-Phe-[D-Trp($NO_2$)]-Lys(Boc)-Thr(But)-Phe-Gaba-OH (Stufe 12.9) durch Cyclisierung mittels DCCI und N-Hydroxybenzotriazol erhalten. Das Rohprodukt wird durch Gegenstromverteilung (K = 1,08) gereinigt.
DC: [Chloroform-Methanol (85:15)] $R_f$ 0,45
[Chloroform-Methanol-Wasser (14:6:1)] $R_f$ 0,60

Beispiel 13

└Asn-Phe-Phe-Trp(Br)]-Lys-Thr-Phe-Gaba┘

(Das Symbol [Trp(Br)] bedeutet 5-Brom-L-tryptophan.) Die Synthese der Titelverbindung erfolgt unter gleichen Bedingungen wie die Synthese des vorangehenden Beispiels 12, jedoch unter Anwendung von 5-Brom-L-tryptophan anstelle des 6-Nitro-D-tryptophans in entsprechenden Ausgangsstoffen und Zwischenprodukten. Gegenstromverteilung: 320 Stufen, K=9,0.

DC: System 101 : $R_f$ 0,60
System 111B : $R_f$ 0,40
112A : $R_f$ 0,52

Der Ausgangsstoff der Formel ⌊Asn-Phe-Phe-[Trp(Br)-Lys(Boc)-Thr(But)-Phe-Gaba⌋ wird folgendermassen erhalten.

*Stufe 13.1*

Bpoc-Phe-[Trp(Br)]-OH

In analoger Weise, wie im Beispiel 12.5A beschrieben, wird 0,57 g 5-Brom-L-tryptophan mit 1,00 g Bpoc-Phe-ONSu umgestezt.

DC: [Chloroform-Methanol (85:15)] $R_f$ 0,15
[Chloroform-Methanol-Wasser (14:6:1)] $R_f$ 0,55

*Stufe 13.2*

Bpoc-Phe[Trp(Br)]-Lys(Boc)-Thr(But)-Phe-Gaba-OTmse

Ein Gemisch von 0,57 g Bpoc-Phe-[Trp(Br)]-OH (Stufe 13.1), 0,63 g H-Lys(Boc)-Thr(But)-Phe-Gaba-OTmse (Beispiel 12.5), 0,13 g N-Hydroxybenzotriazol und 0,21 g DCCI werden in analoger Weise wie bei Beispiel 12.6 umgesetzt. Zur Reinigung wird das Rohprodukt in 10 ml heissem Methanol gelöst und durch Abkühlen ausgefällt.

DC: [Chloroform-Methanol (85:15)] $R_f$ 0,80
[Chloroform-Methanol-Wasser (14:6:1)] $R_f$ 0,90

*Stufe 13.3*

HCl.H-Phe-[Trp(Br)]-Lys(Boc)-Thr(But)-Phe-Gaba-OTmse

Analog Beispiel 12.7 wird 0,84 g Bpoc-Phe-[Trp(Br)]-Lys(Boc)-Thr(But)-Phe-Gaba-OTmse behandelt.

DC: [Chloroform-Methanol (85:15)] $R_f$ 0,65
[Chloroform-Methanol-Wasser (14:6:1)] $R_f$ 0,90

*Stufe 13.4*

SEOC-Asn-Phe-Phe-[Trp(Br)]-Lys(Boc)-Thr(But)-Phe-Gaba-OTmse

Analog Beispiel 12.8 wird 700 mg HCl.H-Phe-[Trp(Br)]-Lys(Boc)-Thr(But)-Phe-Gaba-OTmse (Stufe 13.3) und 250 mg SEOC-Asn-Phe-OH (Beispiel 12.7D) mittels 146 mg DCCI in Gegenwart von 88 mg N-Hydroxybenzotriazol und 60 mg N-Methylmorpholin gekuppelt.

DC: [Chloroform-Methanol (85:15)] $R_f$ 0,77
[Chloroform-Methanol-Wasser (14:6:1)] $R_f$ 0,85

*Stufe 13.5*

H-Asn-Phe-Phe-[Trp(Br)]-Lys(Boc)-Thr(But)-Phe-Gaba-OH

Analog Beispiel 12.9 werden in 500 mg SEOC-Asn-Phe-Phe-[Trp(Br)]-Lys(Boc)-Thr(But)-Phe-Gaba-OTmse (Stufe 13.4) die SEOC- und Tmse-Gruppe gleichzeitig abgespalten.

DC: [Chloroform-Methanol-Wasser (14:6:1)] $R_f$ 0,50

*Stufe 13.6*

⌊Asn-Phe-Phe-[Trp(Br)]-Lys(Boc)-Thr(But)-Phe-Gaba⌋

Durch Cyclisierung vom H-Asn-Phe-Phe-[Trp(Br)]-Lys(Boc)-Thr(But)-Phe-Gaba-OH mittels DCCI und N-Hydroxybenzotriazol gemäss dem im Beispiel 1.10 beschriebenen Verfahren wird ein Rohprodukt erhalten, das durch Gegenstromverteilung (K = 0,86) gereinigt wird.

DC: [Chloroform-Methanol (85:15)] $R_f$ 0,06
[Chloroform-Methanol-Wasser (14:6:1)] $R_f$ 0,85

Beispiel 14

⌊Asn-Phe-Phe-(D-Trp)-Lys-Thr-[Phe(J)]-Gaba⌋

130 mg ⌊Asn-Phe-Phe-(D-Trp)-Lys(Boc)-Thr-[Phe(J)]-Gaba⌋ werden analog Beispiel 1 mit Trifluoressigsäure behandelt und mittels Ionenaustauscher in das Acetat überführt. Die Reinigung erfolgt durch Gegenstromverteilung im gleichen System, wie im Beispiel 1 angegeben; K = 9.

DC: (Kieselgel Merck); System 157C: $R_f$ 0,25

(Das Symbol Ph(J) bedeutet p-Jod-L-phenylalanin.)

Das Ausgangsmaterial wird folgendermassen erhalten.

*Stufe 14.1*

Z-(D-Trp)-Lys(Boc)-Thr-OMe

Eine Lösung von 2,78 g Z-(D-Trp)-Lys(Boc)-OH (Beispiel 1.3A) 0,85 g H-Thr-OMe-Hydrochlorid und 0,77 g N-Hydroxybenzotriazol in 16 ml Acetonitril wird bei —5° mit 0,56 ml N-Methylmorpholin und 1,03 g DCCI versetzt. Nach 15 Stunden bei —5° wird das Gemisch analog Stufe 14.3B aufgearbeitet. Das Produkt wird aus Aethylacetat-Hexan umkristallisiert; Smp.: 120—122°.

DC: System 157A: $R_f$ 0,47

*Stufe 14.1A*

SEOC-Asn-Phe-Phe-OH

Eine Lösung von 2,24 g Z-Asn-Phe-Phe-OH in 40 ml Dimethylformamid wird unter Zusatz von 0,23 g Pd-Kohle (10%) während 4 Stunden bei Raumtemperatur hydriert. Nach dem Abfiltrieren des Katalysators wird die Lösung im Hochvakuum auf ca. 5 ml eingeengt und in dieser Form direkt weiterverarbeitet. Eine Lösung von 1,71 g erhaltenen H-Asn-Phe-Phe-OH in 15 ml Dimethylformamid wird mit 10 ml Wasser versetzt und die erhaltene Suspension nach Zugabe von 1,04 g SEOC-ONSu durch allmähliches Zufügen von 2-N Natronlauge mittels eines automatischen Titriergerätes während 2 Stunden (Raumtemperatur) bei pH von 7,5 gehalten. Nach einer weiteren Zugabe von 1,04 g SEOC-ONSu wird noch während 4 Stunden bei pH 7,5 belassen. Zur Aufarbeitung wird die Lösung von wenig ungelöstem Material abfiltriert, das Filtrat bei 0—5° mit einer dem verbrauchten Natriumhydroxid äquivalenten Menge Salzsäure (ca. 8,0 ml 2-N Lösung) versetzt, das ausgefallene Produkt abfiltriert und getrocknet. Das Rohprodukt wird je einmal aus 10 ml Methanol mit 100 ml Aether und aus 10 ml Methanol mit 80 ml Wasser umgefällt, Smp. 176—182° (Zersetzung).

DC: [Chloroform-Methanol-Wasser (14:6:1)] $R_f$ 0,30

*Stufe 14.2*

SEOC-Asn-Phe-Phe-(D-Trp)-Lys(Boc)-Thr-OMe

Eine Lösung von 340 mg Z-(D-Trp)-Lys(Boc)-Thr-OMe (Stufe 14.1) in einem Gemisch von 15 ml Methanol und Wasser (9:1) wird in Gegenwart von 50 mg Palladium-Kohle (10%) und unter Zugabe von 0,2-N Salzsäure im gleichen Lösungsmittel bei pH 4,5 hydriert. Nach Abfiltrieren des Katalysators wird das Filtrat im Vakuum eingeengt und noch zweimal mit Dimethylformamid eingedampft. Die verbleibende Lösung (total 1,5 g) wird mit 285 mg SEOC-Asn-Phe-Phe-OH (Stufe 14.1A) und 90 mg N-Hydroxybenzotriazol versetzt. Bei —5° werden 0,056 ml N-Methylmorpholin und 123 mg DCCI zugegeben und 18 Stunden bei —5° belassen. Die Aufarbeitung erfolgt analog Beispiel 7.1; das Rohprodukt wird aus wässrigem Trifluoroäthanol umkristallisiert.

DC: System 157A: $R_f$ 0,45

*Stufe 14.3*

SEOC-Asn-Phe-Phe-(D-Trp)-Lys(Boc)-Thr-Hydrazid

Eine Lösung von 355 mg SEOC-Asn-Phe-Phe-(D-Trp)-Lys(Boc)-Thr-OMe (Stufe 14.2) in 1,1 ml Dimethylformamid wird bei Raumtemperatur mit 0,16 ml Hydrazinhydrat versetzt. Nach $2\frac{1}{2}$ Stunden wird die feste Masse mit 5 ml Wasser zerrieben, die Fällung abgenutscht und mit Wasser zum völligen Entfernen von Hydrazin gewaschen. Das nach Zerreiben mit Methanol erhaltene Produkt ist einheitlich.

DC: System 157B: $R_f$ 0,25

*Stufe 14.3A*

Boc-[Phe(J)]-OH

Eine Lösung von 2,9 g p-Jod-L-phenylalanin in 10 ml 1-N Natronlauge und 25 ml tert-Butanol wird mit 2,4 ml Di-tert-butyl-dicarbonat versetzt, und bei Raumtemperatur weitere 3 Stunden gerührt und noch 20 Stunden stehen gelassen. Die Reaktionslösung wird zwischen Wasser und Hexan verteilt; die organische Phase wird verworfen und die wässrige Phase under Eiskühlung angesäuert, das Produkt in Aethylacetat aufgenommen und das Lösungsmittel im Vakuum eingedampft. Das Produkt wird durch Kristallisation aus Tetrachlorkohlenstoff gereinigt; Smp. 118—120°.

DC: System 157B: $R_f$ 0,35

*Stufe 14.3B*

Boc-[Phe(J)]-Gaba-OTmse

Eine Lösung von 406 mg H-Gaba-OTmse (Beispiel 12.2) 782 mg Boc-[Phe(J)]-OH (Stufe 14.3A) und 306 mg N-Hydroxybenzotriazol in 5 ml Acetonitril und 3 ml Dimethylformamid wird bei +5° mit einer Lösung von 412 mg DCCI in 1 ml Dimethylformamid versetzt. Nach 20 Stunden bei 5° wird der ausgefallene Dicyclohexylharnstoff abfiltriert, das Filtrat mit Aethylacetat verdünnt und mit verdünnter Salzsäure und Natriumhydrogencarbonatlösung ausgeschüttelt. Nach Trocknen über Natriumsulfat wird das Lösungsmittel eingedampft und das Produkt aus Hexan umkristallisiert; Smp.: 98—100°.

DC: [Cyclohexan-Aceton (7:3)] $R_f$ 0,35

System 157A $R_f$ 0,68

*Stufe 14.3C*

H-[Phe(J)]-Gaba-OTmse·Hydrochlorid

Eine Lösung von 345 mg Boc-[Phe(J)]-Gaba-OTmse (Stufe 14.3B) in 0,7 ml eines Gemisches von Trifluoräthanol-Wasser (9:1) wird bei Raumtemperatur mit 1,5 ml 1,2-N Salzsäure im gleichen Lösungsmittel versetzt. Nach 30 Minuten wird das Gemisch mit 10 ml tert-Butanol versetzt und im Vakuum auf etwa die Hälfte eingeengt. Dieser Vorgang wird noch dreimal wiederholt, dann das restliche tert-Butanol durch Lyophilisieren entfernt. Der Rückstand wird aus Isopropanol-Aether unkristallisiert.

DC: System 157C: $R_f$ 0,68

*Stufe 14.4*

SEOC-Asn-Phe-Phe-(D-Trp)-Lys(Boc)-Thr-[Phe(J)]-Gaba-OTmse

Eine Lösung von 320 mg SEOC-Asn-Phe-Phe-(D-Trp)-Lys(Boc)-Thr-hydrazid (Stufe 14.3) in 2 ml Dimethylformamid wird bei —15° bis —20° mit 0,14 ml 5,3-N Salzsäure in Dioxan und 0,04 ml tert-Butylnitrit versetzt und 15 Minuten bei dieser Temperatur gerührt. Anschliessend wird bei —25° eine Lösung von 193 mg H-[Phe(J)]-Gaba-OTmse·Hydrochlorid (Stufe 14.3C) in 0,5 ml Dimethylformamid und 0,18 ml N-Methylmorpholin zugefügt, langsam auf 0° erwärmt und 16 Stunden bei dieser Temperatur belassen. Das Produkt wird durch Eintropfen in Wasser gefällt und durch Zerreiben mit Methanol und Acetonitril gereinigt.

DC: [Chloroform-Trifluoräthanol-Methanol (80:15:5)] $R_f$ 0,43

*Stufe 14.5*

H-Asn-Phe-Phe-(D-Trp)-Lys(Boc)-Thr-[Phe(J)]-Gaba-OH

Eine Lösung von 324 mg SEOC-Asn-Phe-Phe-(D-Trp)-Lys(Boc)-Thr-[Phe(J)]-Gaba-OTmse (Stufe 14.4) in 2 ml Dimethylformamid werden mit 12,3 ml 0,34-M Tetraäthylammoniumfluorid in Dimethylsulfoxid versetzt und $1\frac{1}{2}$ Stunden bei 30° belassen. Das Produkt wird durch Eintropfen in 40 ml eiskaltes Wasser, das 0,41 ml 1-N Salzsäure enthält, ausgefällt und nach dem Trocknen in der nächsten Stufe eingesetzt.

DC: System 157: $R_f$ 0,5

*Stufe 14.6*

Asn-Phe-Phe-(D-Trp)-Lys(Boc)-Thr-[Phr(J)]-Gaba

Eine Lösung von 258 mg H-Asn-Phe-Phe-(D-Trp)-Lys(Boc)-Thr-[Phe(J)]-Gaba-OH, 300 mg N-Hydroxybenzotriazol und 410 mg DCCI in 200 ml Dimethylformamid wird 20 Stunden bei 50° belassen. Die Aufarbeitung und Reinigung erfolgt analog Beispiel 8.2; K = 1,1.

DC: System 157A: $R_f$ 0,22

## Beispiel 15

Asn-Phe-Phe-(D-Trp)-Lys-Thr-Tyr(But)-Gaba

Eine Lösung von 350 mg Asn-Phe-Phe-(D-Trp)-Lys(SEOC)-Thr-Tyr(But)-Gaba in 1,2 ml Dimethylformamid wird mit 0,7 ml 2,1-M Tetrabutylammoniumfluorid in Dimethylsulfoxid versetzt und 20 Stunden bei 30° belassen. Nach Versetzen mit 20 ml 1-N Essigsäure und 50 ml Aethylacetat wird die wässrige Phase abgetrennt, die organische Phase noch zweimal mit wenig Wasser extrahiert und die vereinigten wässrigen Lösungen im Vakuum eingeengt und lyophilisiert. Der Rückstand, welcher aus einem Gemisch von Produkt und Tetrabutylammoniumfluorid besteht, wird durch präparative DC auf Kieselgel im System 157C getrennt. Das aus dem Kieselgel isolierte, noch nicht ganz reine Produkt wird durch Gegenstromverteilung im System Chloroform-Tetrachlorkohlenstoff-Methanol 0,05 M Ammoniumacetat (9:1:7:3) über 460 Stufen gereinigt. Das reine Produkt (K = 0,9) wird wie üblich isoliert.

DC (Kieselgel Merck): System 157C: $R_f$ 0,25

Das Ausgangsmaterial wird wie folgt erhalten:

*Stufe 15.1*

Z-(D-Trp)-Lys-OH·Trifluoroacetat

3,4 g Z-(D-Trp)-Lys(Boc)-OH (Beispiel 1.3A) werden unter Eiskühlung in 34 ml einem Gemisch von Trifluoressigsäure-Wasser (9:1), enthaltend 0,5 ml 2-Mercaptoäthanol, eingetragen und, sobald gelöst, noch 40 Minuten bei 23° belassen. Durch Eintropfen in 400 ml Aether und 150 ml Hexan wird das Rohprodukt gefällt und in 30 ml Wasser gelöst. Nach Stehenlassen bei Raumtemperatur während 18 Stunden wird die Lösung lyophilisiert und der Rückstand direkt in die nächste Stufe eingesetzt.

DC: System 157: $R_f$ 0,35

*Stufe 15.2*

Z-(D-Trp)-Lys(SEOC)-OH

Eine Lösung von 3,0 g Z-(D-Trp)-Lys-OH-Trifluoracetat (Stufe 15.1) und 1,47 g (2-Trimethylsilyläthyl)-(N-Hydroxy)-succinimidocarbonat (Beispiel 12.7A) in 5,2 ml Dimethylformamid wird bei Raumtemperatur mit 0,87 ml Triäthylamin versetzt und 15 Stunden stehen gelassen. Nach Verdünnen mit Aethylacetat wird mit Salzsäure auf pH 1-2 angesäuert, die Aethylacetat-Lösung mit Wasser gewaschen, über Natriumsulfat getrocknet und eingedampft. Das Rohprodukt wird durch Gegenstromverteilung über 140 Stufen im gleichen System, wie im Beispiel 1.10 beschrieben, gereinigt; K = 0,75.
DC: System 157C: $R_f$ 0,55

*Stufe 15.3*
Z-(D-Trp)-Lys(SEOC)-Thr-OMe

Eine Lösung von 3,16 g Z-(D-Trp)-Lys(SEOC)-OH (Stufe 15.2), 1,32 g H-Thr-OMe·Hydrochlorid und 0,95 g N-Hydroxybenzotriazol in 25 ml Acetonitril wird bei —5° mit 0,86 ml N-Methylmorpholin und 1,34 g DCCI versetzt und 16 Stunden bei dieser Temperatur belassen. Das nach Abfiltrieren des Dicyclohexylharnstoffs erhaltene Filtrat wird mit Aethylacetat verdünnt, mit verdünnter Salzsäure und einer Natriumhydrogencarbonatlösung gewaschen und über Natriumsulfat getrocknet. Der nach Eindampfen des Lösungsmittels erhaltene Rückstand wird mit Gemischen von Aethylacetat-Hexan (7:3) bis (9:1) über eine Kieselgelsäule chromatographiert. Die dünnschichtchromatographisch reinen Fraktionen werden zur Trockne eingedampft.
DC: System 157A: $R_f$ 0,38

*Stufe 15.4*
Z-Asn-Phe-Phe-(D-Trp)-Lys(SEOC)-Thr-OMe

Eine Lösung von 1,31 g Z-(D-Trp)-Lys(Boc)-Thr-OMe in 30 ml Methanol-Wasser (95:5) wird über 130 mg Palladium-Kohle (10%) während 2 Stunden hydriert. Nach Abfiltrieren des Katalysators wird das Filtrat eingeengt und zweimal mit Dimethylformamid im Hochvakuum eingedampft. Die erhaltene Lösung, welche 3,5 ml Dimethylformamid enthält, wird mit 1,01 g Z-Asn-Phe-Phe-OH (Beispiel 1.5A) und 330 mg N-Hydroxybenzotriazol versetzt, auf —5° abgekühlt, mit einer Lösung von 440 mg DCCI in 0,5 ml Dimethylformamid behandelt, 15 Stunden bei 0° stehen gelassen und analog Beispiel 7.1 aufgearbeitet.
DC: System 157A: $R_f$ 0,45

*Stufe 15.5*
Z-Asn-Phe-Phe-(D-Trp)-Lys(SEOC)-Thr-hydrazid

Eine Lösung von 2,0 g Z-Asn-Phe-Phe-(D-Trp)-Lys(SEOC)-Thr-OMe in 6 ml Dimethylformamid wird bei Raumtemperatur mit 0,85 ml Hydrazinhydrat versetzt. Nach 2,15 Stunden wird wie im Beispiel 14.3 aufgearbeitet. Das Produkt kann wegen seiner ausserordentlichen Schwerlöslichkeit nicht durch DC kontrolliert werden.

*Stufe 15.5A*
Trt-Tyr(But)-OH·Diäthylammoniumsalz

Eine Lösung von 4,0 g H-Tyr(But)-OH in 50 ml Chloroform wird mit 6,7 ml Wasser, 6,7 ml Diäthylamin und unter Eiskühlung innert 30 Minuten mit 7,05 g Triphenylchlormethan versetzt. Nach weiteren 1½ Stunden wird die wässrige Phase abgetrennt, die Chloroformlösung je zweimal mit einer 4%igen wässrigen Diäthylaminlösung und gesättigter Kochsalzlösung ausgeschüttelt, über Natriumsulfat getrocknet und vom Lösungsmittel im Vakuum befreit. Der Rückstand wird aus Aether-Petroläther kristallisiert; Smp. 141—145°.
DC: [Toluol-Aceton (1:1)] $R_f$ 0,48

*Stufe 15.5B*
Trt-Tyr(But)-ONSu

4,7g Trt-Tyr(But)-OH-Diäthylammoniumsalz (Stufe 15.5A) werden unter Eiskühlung mit 100 ml Aethylacetat und je 10 ml 0,5-M Kaliumsulfat- und Kaliumhydrogensulfat-Lösung geschüttelt. Die Aethylacetat-Lösung wird nach Abtrennen der unteren Phase mit Wasser gewaschen und mit Natriumsulfat getrocknet, und das Lösungsmittel schonend im Vakuum abdestilliert. Der Rückstand wird in 50 ml Dimethylformamid gelöst, mit 1,1 g N-Hydroxysuccinimid und bei 0° mit 1,94 g DCCI versetzt und 15 Stunden bei +5° belassen. Nach Abfiltrieren des Dicyclohexylharnstoffs wird aus dem Filtrat mit Wasser ein Rohprodukt gefällt, das an einer Kieselgel-Säule mit Chloroform chromatographiert wird. Die Fraktionen, die das Produkt gemäss DC enthalten, werden vereinigt und aus Methanol umkristallisiert; Smp. 163—164°.
DC: [Chloroform-Aethylacetat (1:1)] $R_f$ 0,53

*Stufe 15.5C*
Trt-Tyr(But)-Gaba-OBzl

Zu einer Suspension von 580 mg Trt-Tyr(But)-ONSu (Stufe 15.5B) und 730 mg H-Gaba-OBzl·p-Toluolsulfonat (Beispiel 1.7A) in 1 ml reinem Chloroform werden 0,77 ml N-Methylmorpholin gegeben

und 4 Tage bei Raumtemperatur belassen. Nach Verdünnen mit Aethylacetat wird die Lösung unter Eiskühlung mit 0,2 M Kaliumhydrogensulfatlösung und Wasser ausgeschüttelt, die organische Phase über Natriumsulfat getrocknet, und das Lösungsmittel abdestilliert. Das Rohprodukt wird an Kieselgel chromatographiert. Das reine Produkt wird mit einem Gemisch von Aethylacetat-Hexan (1:1) eluiert und wie üblich isoliert.

DC: [Toluol-Aceton (8:2)] $R_f$ 0,5

*Stufe 15.5D*

H-Tyr(But)-Gaba-OBzl·Hydrochlorid

Eine Lösung von 620 mg Trt-Tyr(But)-Gaba-OBzl (Stufe 15.5C) in 20 ml einem Gemisch von Trifluoräthanol-Wasser (9:1) wird mittels eines automatischen Tiltriergerätes bei pH 3,5 mit 1,2-N Salzsäure im gleichen Lösungsmittel versetzt bis kein Verbrauch mehr stattfindet. Nach Zugabe von 20 ml tert-Butanol wird im Vakuum eingeengt. Dies wird noch zweimal wiederholt, und das restliche tert-Butanol durch Lyophilisieren entfernt. Zur Entfernung von Triphenylcarbinol wird das Lyophilisat zwischen Wasser und Aether verteilt, die wässrige Lösung wird nachher lyophilisiert.

DC: System 157B $R_f$ 0,5

*Stufe 15.6*

Z-Asn-Phe-Phe-(D-Trp)-Lys(SEOC)-Thr-Tyr(But)-Gaba-OBzl

Eine Lösung von 975 mg Z-Asn-Phe-Phe-(D-Trp)-Lys(SEOC)-Thr-hydrazid (Stufe 15.5) in 7 ml Dimethylformamid wird bei —15° bis —20° mit 0,47 ml 4,6 N Salzsäure in Dioxan und 0,11 ml tert-Butylnitrit versetzt. Nach 15 Minuten wird zu diesem Reaktionsgemisch bei —25° eine Lösung von 385 mg H-Tyr(But)-Gaba-OBzl·Hydrochlorid (Stufe 15.5D) in 0,5 ml Dimethylformamid und 0,44 ml N-Methylmorpholin zugegeben und das Gemisch 15 Stunden bei 0° stehen gelassen. Das Produkt wird mit Wasser gefällt und durch Zerreiben mit Acetonitril und Methanol gereinigt. Wegen Unlöslichkeit des Produktes kann keine DC-Kontrolle erfolgen.

*Stufe 15.7*

H-Asn-Phe-Phe-(D-Trp)-Lys(SEOC)-Thr-Tyr(But)-Gaba-OH

Eine Lösung von 1,1 g Z-Asn-Phe-Phe-(D-Trp)-Lys(SEOC)-Thr-Tyr(But)-Gaba-OBzl in 30 ml Dimethylformamid wird in Gegenwart von 100 mg Palladium-Kohle (10%) während 3 Stunden hydriert. Der Katalystor wird abgenutscht, das Filtrat eingeengt und das Produkt mit Wasser gefällt. Gereinigt wird durch Lösen in 5 ml Dimethylformamid und Fällen mit 15 ml Methanol.

DC: System 157: $R_f$ 0,55

*Stufe 15.8*

└Asn-Phe-Phe-(D-Trp)-Lys(SEOC)-Thr-Tyr(But)-Gaba┘

Eine Lösung von 700 mg H-Asn-Phe-Phe-(D-Trp)-Lys(SEOC)-Thr-Tyr(But)-Gaba-OH (Stufe 15.7), N-Hydroxybenzotriazol und 1,11 g DCCI in 540 ml Dimethylformamid wird während 21 Stunden bei 50° cyclisiert. Die Aufarbeitung erfolgt analog Beispiel 7.3. Gereinigt wird durch Säulenchromatographie über Kieselgel, die reine Substanz wird mit einem Gemisch von Chloroform-Trifluoräthanol-Methanol (87:5:8) eluiert.

DC: System 157B: $R_f$ 0,38

Beispiel 16

A) Eine Injektionslösung enthaltend 2,0 mg des gemäss Beispiel 1 erhaltenen Oktapeptids:

└Asn-Phe-Phe-(D-Trp)-Lys-Thr-Phe-Gaba┘

(weiterhin als "Wirkstoff" bezeichnet) wird folgendermassen erhalten: Man löst 1,0 mg Eisessig, 0,8 mg Natriumacetat, 8,0 mg Natriumchlorid und 2,0 mg Wirkstoff in 0,7 ml destilliertem Wasser und füllt mit destilliertem Wasser auf 1 ml auf. Die Lösung wird bei 120°C 20 Minuten lang im Autoklav erhitzt. Das pH nach der Sterilisation ist 4,5.

B) Eine Injektionslösung enthaltend 0,5 mg des Wirkstoffes wird folgendermassen erhalten:

Man löst in 0,7 ml physiologischer Natriumchloridlösung 0,5 mg Wirkstoff und säuert die Lösung mit 0,1-N Salzsäure auf pH 4,0 an. Mit destilliertem Wasser wird auf 1 ml aufgefüllt und sterilfiltriert.

In gleicher Weise werden als Wirkstoff die in Beispielen 2 bis 15 beschriebenen Endstoffe angewendet.

Beispiel 17

A) Eine Gelatine-enthaltende Injektionslösung enthaltend 0,1 mg Wirkstoff (vgl. Beispiel 16) wird folgendermassen erhalten:

Eine sterilfiltrierte wässrige Lösung des Wirkstoffes wird unter Erwärmen mit einer sterilen Gelatinelösung, welche als Konservierungsmittel Phenol enthält, unter aseptischen Bedingungen vermischt, sodass 1,0 ml Lösung folgende Zusammensetzung hat:

| | |
|---|---|
| Wirkstoff | 0,1 mg |
| Gelatine | 150,0 mg |
| Phenol | 4,7 mg |
| dest. Wasser bis zu | 1,0 ml |

Die Mischung wird aseptisch in Vials zu 1,0 ml abgefüllt.

B) Eine analoge Injektionslösung enthaltend 0,5 mg des Wirkstoffes erhält man in gleicher Weise, wie oben angegeben, indem man eine Mischung der folgenden Zusammensetzung herstellt:

| | |
|---|---|
| Wirkstoff | 0,5 mg |
| Gelatine | 280,0 mg |
| Phenol | 5,0 mg |
| dest. Wasser bis zu | 1,0 ml |

Die Mischung wird aseptisch in Vials zu 1,0 ml abgefüllt.

Beispiel 18

Präparat enthaltend 0,5 mg Wirkstoff (vgl. Beispiel 16) als sterile Trockensubstanz zur Injektion wird folgendermassen erhalten:

Man löst 0,5 mg Wirkstoff in 1 ml einer wässrigen Lösung von 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in destilliertem Wasser gelöst. Die Lösung wird intramuskulär oder intravenös angewendet.

Beispiel 19

Injektionspräparat enthaltend den Wirkstoff (vgl. Beispiel 16) als Polyphosphat-Suspension wird folgendermassen erhalten:

A) Mit 1,0 mg Wirkstoff;

Man mischt eine Lösung von 1,0 mg Wirkstoff und 9,0 mg Natriumchlorid in 0,5 ml destilliertem Wasser mit einer Lösung von 2 mg Natriumpolyphosphat (Calgon® N) in 0,5 ml destilliertem Wasser. Die erhaltene Suspension hat folgende Zusammensetzung:

| | |
|---|---|
| Wirkstoff | 1,0 mg |
| Natriumpolyphosphat (Calgon® N) | 2,0 mg |
| Natriumchlorid | 9,0 mg |
| dest. Wasser bis zu | 1,0 ml |

Die Suspension weist ein pH von 6,9 auf. Sie ist für intramuskuläre Anwendung geeignet.

B) Mit 0,5 mg Wirkstoff:

In gleicher Weise wie oben angegeben wird eine Suspension der folgenden Zusammensetzung hergestellt.

| | |
|---|---|
| Wirkstoff | 0,5 mg |
| Natriumpolyphosphat (Calgon® 322) | 1,0 mg |
| Natriumchlorid | 9,0 mg |
| dest. Wasser bis zu | 1,0 ml |

Das pH der Suspension beträgt 5,9.

Beispiel 20

Injektionspräparat enthaltend 0,3 mg Wirkstoff (vgl. Beispiel 16) in Form eines oeligen Aluminiumstearat-Gel.

Ein 2%iges Aluminiumstearat-Gel wird auf übliche Art und Weise durch Suspendieren von 1,0 g Aluminiummonostearat in 49,0 g Erdnussöl und anschliessendes Erwärmen auf 130°C während 10 Min. hergestellt. 15,0 mg Wirkstoff werden mit 0,3 g obigem Aluminiumstearat-Gel suspendiert, homogenisiert und mit der restlichen Menge Aluminiumstearat-Gel verdünnt. Das so erhaltene Gel hat folgende Zusammen setzung:

| | |
|---|---|
| Wirkstoff | 0,3 mg |
| Aluminiummonostearat | 20,0 mg |
| Erdnussöl bis zu | 1,0 mg |

Die ölige Aluminiumstearat-Gel-Suspension ist für intramuskuläre Anwendung geeignet.

Beispiel 21

Injektionspräparat enthaltend 0,5 mg Wirkstoff (vgl. Beispiel 16) als eine Depot-Suspension mit Dextransulfat.

Man löst in 0,4 ml destilliertem Wasser 0,36 mg Essigsäure, 1,9 mg Natriumacetat-trihydrat, 0,8 mg Natriumchlorid und 0,5 mg Wirkstoff und füllt mit destilliertem Wasser auf 0,5 ml auf. Zu dieser Lösung wird unter Rühren 0,5 ml einer 0,1%igen Lösung von Dextransulfat (Molekulargewicht 500'000) gegeben, womit sich eine homogene Ausfällung bildet. Die erhaltene Suspension hat folgende Zusammensetzung:

| | |
|---|---|
| Wirkstoff | 0,50 mg |
| Dextransulfat MG 500'000 | 0,50 mg |
| Essigsäure 100% | 0,36 mg |
| Natriumacetat-trihydrat | 1,90 mg |
| Natriumchlorid | 8,00 mg |
| dest. Wasser bis zu | 1,00 ml |

Die wässrige Suspension ist für intramuskuläre und subcutane Injektion geeignet.

Beispiel 22

*Nasal-Spray:*

30 mg feingemahlener Wirkstoff (vgl. Beispiel 16) wird in einer Mischung von 75 mg Benzylalkohol und 1,395 g eines Gemisches von halbsynthetischen Glyceriden gesättigter Fettsäuren mit 8—12 C-Atomen (z.B. Miglyol® 812) suspendiert. Mit dieser Suspension werden Aluminium-Monoblocdosen (Gehalt 10 ml) eingefüllt, mit einem Dosierventil verschlossen und mit 6,0 g eines Gemisches von 40 Volumteilen von Dichlordifluormethan und 60 Volumteilen von 1,2-Dichlor-1,1,2,2-tetrafluoräthan (Freon® 12/114) unter Stickstoffdruck eingefüllt. Die Aluminiumdose mit einem Füllgewicht von insgesamt 7,5 g enthält 100 Einzeldosen à 0,3 mg Wirkstoff. Die Sprühdose ist mittels des Ventils so eingestellt, dass bei einmaligem Druck eine Einzeldosis versprüht wird.

In gleicher Weise werden Nasensprays hergestellt, welche statt Miglyol® die gleiche Menge Isopropylmyristat oder Isopropylpalmitat oder einer Mischung von Glycerin- und Polyoxyaethylenglycolestern von Fettsäuren mit 8 und 10 Kohlenstoffatomen Labrafac® WL 1219) enthalten.

**Patentansprüche**

1. Verfahren zur Herstellung von Cyclopeptiden der Formel

$$\overline{\underline{\text{R-Phe-Phe-trp-Lys-Thr-Phe(W)-X -Y}}}$$
$$\text{5 6 7 8 9 10 11 12 13} \qquad (I)$$

worin R für Asn, Ala oder des-R, trp für D-Trp oder L-Trp, das im Benzolring durch Halogenatome oder Nitrogruppen substituiert sein kann, W für eine am Benzolring des L-Phenylalaninrests als Substituent befindliche gegebenenfalls verätherte Hydroxylgruppe oder Halogenatom, oder für Wasserstoff, X für

den Rest einer $\omega$-Aminoniederalkan-(mono oder di)-carbonsäure oder des-X und Y für den Rest einer $\omega$-Aminoniederalkan-(mono oder di)-carbonsäure oder des-Y steht, mit Ausnahme einer Verbindung, worin R für des-R, trp für D-Trp oder L-Trp, W für Wasserstoff, eines der Symbole X und Y für des-X bzw. des-Y und das andere für den Rest der 7-Aminoheptansäure steht, sowie von Säureadditionssalzen und Komplexen davon, dadurch gekennzeichnet, dass man aus einer Verbindung der Formel

R-Phe-Phe-trp-Lys(A)-Thr(B)-Phe(W)-X-Y (cyclisch) (II)

worin R, W, X, Y und trp die obgenannten Bedeutungen haben, A eine $\varepsilon$-Aminoschutzgruppe oder Wasserstoff und B eine Hydroxylschutzgruppe oder Wasserstoff bedeutet, wobei nur eines der Symbole A und B für Wasserstoff stehen kann, die Schutzgruppe(n) abspaltet, und wenn erwünscht, aus einem erhaltenen Säureadditionssalz die entsprechende Base freisetzt und/oder eine erhaltene Base in ein Säureadditionssalz davon umwandelt.

2. Verfahren gemäss Anspruch 1, dadurch gekennzeichnet, dass man ein lineares Peptid der Formel

$$H—[II']—C \quad (III)$$

worin II' einen der im Anspruch 1 definierten Formel II entsprechenden Rest darstellt, in welchem die amidische Bindung zwischen zwei beliebigen benachbarten Aminosäure-Resten des Peptidringes unterbrochen ist, und C für eine freie Hydroxylgruppe, eine durch eine Aktivierungsgruppe abgewandelte Hydroxylgruppe oder die Hydrazinogruppe $—NH—NH_2$ steht, unter Schutz der $\varepsilon$-Aminogruppe des Lysin-Rests cyclisiert und anschliessend die Schutzgruppe(n) abspaltet, und wenn erwünscht, aus einem erhaltenen Säureadditionssalz die entsprechende Base freisetzt und/oder eine erhaltene Base in ein Säureadditionssalz davon umwandelt.

3. Cyclopeptide der Formel

R-Phe-Phe-trp-Lys-Thr-Phe(W)-X -Y (cyclisch)
5 6 7 8 9 10 11 12 13 (I)

worin R für Asn, Ala oder des-R, trp für D-Trp oder L-Trp, das im Benzolring durch Halogenatome oder Nitrogruppen substituiert sein kann, W für eine am Benzolring des L-Phenylalaninrests als Substituent befindliche gegebenenfalls verätherte Hydroxylgruppe oder Halogenatom, oder für Wasserstoff, X für den Rest einer $\omega$-Aminoniederalkan-(mono oder di)-carbonsäure oder des-X und Y für den Rest einer $\omega$-Aminoniederalkan-(mono oder di)-carbonsäure oder des-Y steht, siche Anspruch 1 sowie Säureadditionssalze und Komplexe davon.

4. Eine Verbindung gemäss Anspruch 3 der Formel I, worin R für Asn oder des-R, trp für D-Trp oder L-Trp, W für Wasserstoff, X für den Rest einer $\omega$-Aminoniederalkanmonocarbonsäure oder des-X, und Y für den Rest einer $\omega$-Aminoniederalkanmonocarbonsäure oder des-Y steht, mit Ausnahme einer Verbindung, worin R für des-R, einer der Symbole X und Y für des-X bzw. des-Y und das andere für den Rest den 7-Aminoheptansäure steht, oder ein Säureadditionssalz oder ein Komplex davon.

5. Eine Verbindung gemäss Anspruch 4, worin der Rest einer $\omega$-Aminoniederalkanmonocarbonsäure durch die Formel

$$—NH—(CH_2)_n—CO—$$

worin n eine ganze Zahl von 1 bis 7 bedeutet, charakterisiert ist.

6. Eine Verbindung gemäss Anspruch 3, worin der Rest einer $\omega$-Aminoniederalkandicarbonsäure durch die Formel

$$—NH—\underset{\displaystyle COOH}{\underset{|}{CH}}—(CH_2)_{n-1}—CO—$$

worin n eine ganze Zahl von 1 bis 7 bedeutet, charakterisiert ist.

7. Eine Verbindung gemäss Anspruch 4, worin der Tryptophan$^8$-Rest die D-Konfiguration hat und —X—Y— zusammen $—[Gaba]_p—$, worin p gleich 0, 1 oder 2 ist, bedeutet.

8. Eine Verbindung gemäss Anspruch 7, charakterisiert durch die Formel

Asn-Phe-Phe-[D-trp]-Lys-Thr-Phe-Gaba (cyclisch)

9. Eine Verbindung gemäss Anspruch 7, charakterisiert durch die Formel

Asn-Phe-Phe-[D-trp]-Lys-Thr-Phe-Gaba-Gaba (cyclisch)

10. Eine Verbindung gemäss einem der Ansprüche 3 bis 10 in Form eines therapeutisch anwendbaren Säureadditionssalzes oder Komplexes.

11. Ein pharmazeutisches Präparat gekennzeichnet durch den Gehalt von mindestens einer der in den Ansprüchen 3 bis 10 characterisierten Verbindungen.

12. Eine in einem der Ansprüche 3 bis 10 characterisierte Verbindung zur Verwendung für therapeutische Behandlung der übermässigen Sekretion von Somatotropin, Insulin und/oder Glucagon.

**Revendications**

1. Procédé de préparation de cyclopeptides de formule

$$\overline{\text{R-Phe-Phe-trp-Lys-Thr-Phe(W)-X -Y}} \quad (I)$$
5 6 7 8 9 10 11 12 13

où R représente Asn, Ala ou des-R, trp représente D-Trp ou L-Trp, qui peut être substitué dans le noyau benzène par des atomes d'halogène ou des groupes nitro, W un groupe hydroxyle éventuellement éthérifié se trouvant comme substituant sur le noyau benzène du radical L-phénylalanine ou un atome d'halogène, ou un hydrogène, X le radical d'un acide $\omega$-aminoalcane inférieur-(mono- ou di)-carboxylique ou des-X et Y le radical d'un acide $\omega$-aminomonoalcane inférieur-(mono- ou di)-carboxylique ou des-Y, à l'exception d'un composé où R représente des-R, trp D-Trp ou L-Trp, W un hydrogène, l'un des symboles X et Y des-X ou des-Y et l'autre le radical de l'acide 7-aminoheptanoïque, ainsi que de leurs sels d'addition acides et complexes, caractérisé en ce qu'on sépare le(s) groupe(s) protecteur(s) dans un composé de formule

$$\overline{\text{R-Phe-Phe-trp-Lys(A)-Thr(B)-Phe(W)-X-Y}} \quad (II)$$

où R, W, X, Y et trp ont les significations données ci-dessus, A représente un groupe protecteur d'$\varepsilon$-amino ou un hydrogène et B un groupe protecteur d'hydroxyle ou un hydrogène, où seul un des symboles A et B peut représenter un hydrogène, et, si on le désire, en ce qu'on libère la base correspondante à partir d'un sel d'addition acide obtenu et/ou en ce qu'on transforme une base obtenue en un de ses sels d'addition acides.

2. Procédé selon la rev. 1, caractérisé en ce qu'on cyclise un peptide linéaire de formule

$$\text{H—(II')—C} \quad (III)$$

où II' représente un radical correspondant à la formule II définie dans la revendication 1, où la liaison amidique est interrompue entre deux radicaux acide aminé voisins quelconques du noyau peptidique, et C représente un groupe hydroxyle libre, un groupe hydroxyle modifié par un groupe activant ou le groupe hydrazino —NH—$NH_2$, sous la protection du groupe $\varepsilon$-amino du radical lysine, puis en ce qu'on sépare le(s) groupe(s) protecteur(s) et, si on le désire, en ce qu'on libère à partir d'un sel d'addition acide obtenu la base correspondante et/ou ce qu'on transforme une base obtenue en un de ses sels d'addition acides.

3. Cyclopeptides de formule

$$\overline{\text{R-Phe-Phe-trp-Lys-Thr-Phe(W)-X -Y}} \quad (I)$$
5 6 7 8 9 10 11 12 13

où R représente Asn, Ala ou des-R, trp D-Trp ou L-Trp, qui peut être substitué dans le noyau benzène par des atomes d'halogène ou des groupes nitro, W un groupe hydroxyle éventuellement éthérifié se trouvant comme substituant sur le noyau benzène du radical L-phénylalanine ou un atome d'halogène, ou un hydrogène, X le radical d'un acide $\omega$-aminoalcane inférieur-(mono- ou di)-carboxylique ou des-X et Y le radical d'un acide $\omega$-aminoalcane inférieur-(mono ou di)-carboxylique ou des-Y, à l'exception d'un composé où R représente des-R, trp D-Trp ou L-Trp, W un hydrogène, l'un des symboles X et Y des-X ou des-Y et l'autre le radical de l'acide 7-aminoheptanoïque, ainsi que leurs sels d'addition acides et complexes.

4. Composé selon la revendication 3 de formule I, où R représente Asn ou des-R, trp D-Trp ou L-Trp, W un hydrogène, X le radical d'un acide $\omega$-aminoalcane inférieur-monocarboxylique ou des-X, et Y le radical d'un acide $\omega$-aminoalcane inférieur-monocarboxylique ou des-Y, à l'exception d'un composé où R représente des-R, l'un des symboles X et Y des-X ou des-Y et l'autre le radical de l'acide 7-aminoheptanoïque, ou un de ses sels d'addition acides ou complexes.

5. Composé selon la revendication 4, où le radical d'un acide $\omega$-aminoalcane inférieur-monocarboxylique est caractérisé par la formule

$$\text{—NH—}(CH_2)_n\text{—CO—}$$

où n est un nombre entier allant de 1 à 7.

6. Composé selon la revendication 3, où le radical d'un acide ω-aminoalcane inférieur-dicarboxylique est caractérisé par la formule

$$-NH-\underset{\displaystyle COOH}{\underset{|}{CH}}-(CH_2)_{n-1}-CO\grave{e}$$

où n représente un nombre entier allant de 1 à 7.

7. Composé selon la revendication 4, où le radical tryptophane[8] a la configuration D et où —X—Y— représentent ensemble —$(Gaba)_p$—, où p vaut 0, 1 ou 2.

8. Composé selon la revendication 7, caractérisé par la formule

└Asn-Phe-Phe-(D-trp)-Lys-Thr-Phe-Gaba┘

9. Composé selon la revendication 7, caractérisé par la formule

└Asn-Phe-Phe-(D-trp)-Lys-Thr-Phe-Gaba-Gaba┘

10. Composé selon l'une des revendications 3 à 10 sous la forme d'un sel d'addition acide ou d'un complexe thérapeutiquement acceptable.

11. Préparation pharmaceutique caractérisée en ce qu'elle contient au moins l'un des composés caractérisés dans les revendications 3 à 10.

12. Composé caractérisé dans les revendications 3 à 10 aux fins d'utilisation pour le traitement thérapeutique de la sécrétion excessive de somatotropine, d'insuline et/ou de glucagon.

**Claims**

1. A process for the preparation of a cyclopeptide of the formula

└R-Phe-Phe-trp-Lys-Thr-Phe(W)-X -Y ┘ (I)
5 6 7 8 9 10 11 12 13

in which R is Asn, Ala or de-R, trp is D-Trp or L-Trp, which can be substituted in the benzene ring by halogen atoms or nitro groups, W is a free or etherified hydroxyl group or halogen atom present as a substituent on the benzene ring of the L-phenylalanine radical, or is hydrogen, X is the radical of an ω-amino-lower alkane-(mono or di)-carboxylic acid or de-X and Y is the radical of an ω-amino-lower alkane-(mono or di)-carboxylic acid or de-Y, with the exception of a compound wherein R is de-R, trp is D-Trp or L-Trp, W is hydrogen, one of the symbols X and Y is de-X or de-Y and the other is the radical of 7-aminoheptanoic acid, or of an acid addition salt or complex thereof, which comprises detaching the protective group or groups from a compound of the formula

└R-Phe-Phe-trp-Lys(A)-Thr(B)-Phe(W)-X-Y┘ (II)

in which R, W, X, Y and trp are as defined above, A is an ε-amino protective group or hydrogen and B is a hydroxyl protective group or hydrogen, it being possible for only one of the symbols A and B to be hydrogen, and, if desired, liberating the corresponding base from a resulting acid addition salt and/or converting a resulting base into an acid addition salt thereof.

2. A process according to claim 1, which comprises cyclising a linear peptide of the formula

H—[II′]—C (III)

in which II′ is a radical corresponding to the formula II defined in Claim 1, in which the amide bond between any two adjacent amino acid radicals of the peptide ring is interrupted, and C is a free hydroxyl group, a hydroxyl group modified by an activating group, or the hydrazino group —NH—$NH_2$, the ε-amino group of the lysine radical being protected, and subsequently detaching the protective group or groups, and, if desired, liberating the corresponding base from a resulting acid addition salt and/or converting a resulting base into an acid addition salt thereof.

3. A cyclopeptide of the formula

└R-Phe-Phe-trp-Lys-Thr-Phe(W)-X -Y ┘ (I)
5 6 7 8 9 10 11 12 13

wherein R is Asn, Ala or de-R, trp is D-Trp or L-Trp, which can be substituted in the benzene ring by halogen atoms or nitro groups, W is a free or etherified hydroxyl group or halogen atom present as a

substituent on the benzene ring of the L-phenylalanine radical, or is hydrogen, X is the radical of an $\omega$-amino-lower-alkane-(mono or di)-carboxylic acid or de-X, and Y is the radical of a $\omega$-amino-lower-alkane(mono or di)-carboxylic acid or de-Y, with the exception of a compound wherein R is de-R, trp is D-Trp or L-Trp, W is hydrogen, one of the symbols X and Y is de-X or de-Y and the other is the radical of 7-aminoheptanoic acid, or an acid addition salt or complex thereof.

4. A compound according to Claim 3 of the formula I, wherein R is Asn or de-R, trp is D-Trp or L-Trp, W is hydrogen, X is the radical of an $\omega$-amino-lower-alkane-monocarboxylic acid or de-X, and Y is the radical of an $\omega$-amino-lower-alkanemonocarboxylic acid or de-Y, with the exception of a compound wherein R is de-R, one of the symbols X and Y is de-X or de-Y and the other is the radical of 7-aminoheptanoic acid, or an acid addition salt or complex thereof.

5. A compound according to Claim 4, wherein the radical of an $\omega$-amino-lower-alkanemonocarboxylic acid is characterised by the formula

$$—NH—(CH_2)_n—CO—$$

wherein n is an integer from 1 to 7.

6. A compound according to Claim 3, wherein the radical of an $\omega$-amino-lower-alkane-dicarboxylic acid is characterised by the formula

$$—NH—\underset{\displaystyle COOH}{\underset{|}{CH}}—(CH_2)_{n-1}—CO—$$

wherein n is an integer from 1 to 7.

7. A compound according to Claim 4, wherein the tryptophan[8] radical has the D configuration, and —X—Y— together are —$[Gaba]_p$—, wherein p is nought, 1 or 2.

8. A compound according to Claim 7, characterised by the formula

$$\boxed{\text{Asn-Phe-Phe-[D-trp]-Lys-Thr-Phe-Gaba}}$$

9. A compound according to Claim 7, characterised by the formula

$$\boxed{\text{Asn-Phe-Phe-[D-trp]-Lys-Thr-Phe-Gaba-Gaba}}$$

10. A compound according to any one of claims 3 to 10 in the form of a therapeutically applicable acid addition salt or complex.

11. A pharmaceutical preparation, characterised by the content of at least one of the compounds of claims 3 to 10.

12. A compound characterised in any one of claims 3 to 10 for use in the therapeutic treatment of excessive secretion of somatotropin, insulin and/or glucagon.